# EUROPÄISCHE PATENTSCHRIFT

(11) **EP 4 015 498 B1**
(45) Veröffentlichungstag und Bekanntmachung des Hinweises auf die Patenterteilung: **24.04.2024**
(21) Anmeldenummer: 20215512.3
(22) Anmeldetag: 18.12.2020
(51) Int. Cl.: C07C 67/08, C07C 67/54, C07C 67/58, C07C 69/54

(54) **VERFAHREN ZUR KONTINUIERLICHEN HERSTELLUNG VON ACRYLSÄURE-N-BUTYLESTER**
METHOD FOR THE CONTINUOUS PRODUCTION OF ACRYLIC ACID N-BUTYLESTER
PROCÉDÉ DE PRODUCTION CONTINUE D'ACIDE ACRYLIQUE-N-BUTYLESTER

(43) Veröffentlichungstag der Anmeldung: 22.06.2022
(73) Patentinhaber: BASF SE, 67056 Ludwigshafen am Rhein (DE)
(72) Erfinder: MAKARCZYK, Piotr, 67056 Ludwigshafen (DE); AKIN, Aykan, 200137 Shanghai (CN); LANG, Ortmund, 67056 Ludwigshafen (DE); HOFFMANN, Michael, 67056 Ludwigshafen (DE); SCHLIEPHAKE, Volker, 67105 Schifferstadt (DE)
(74) Vertreter: BASF IP Association

(56) Entgegenhaltungen:
- DE-A1- 19 851 983
- DE-A1- 19 935 453

## Beschreibung

Die vorliegende Erfindung betrifft ein Verfahren zur kontinuierlichen Herstellung von Acrylsäure-n-butylester durch Umsetzung von Acrylsäure mit n-Butanol in an Lösungsmittel freier Phase bei erhöhter Temperatur und unter Zusatz von Säure als saurem Veresterungskatalysator, bei dem man die Acrylsäure, das n-Butanol und den Veresterungskatalysator einer Reaktionszone zuführt, während einer Verweilzeit in der Reaktionszone das gebildete Wasser als Bestandteil eines n-Butanol umfassenden Gemisches in einer der Reaktionszone aufgesetzten ersten Rektifikationseinheit I vom Reaktionsgemisch abtrennt, das dabei anfallende Destillat in eine n-Butanol enthaltende organische und in eine Wasser enthaltende wässrige Phase auftrennt, die organische Phase in die Rektifikationseinheit I zurückführt, die wässrige Phase gegebenenfalls, ganz oder teilweise, einer Strippeinheit IX zuführt, das den Acrylsäure-n-butylester enthaltende Reaktionsgemisch, das aus der Reaktionszone abgezogen wird, einer Vorreinigung zuführt, wobei
1. in einer ersten Vorreinigungsstufe (Vorreinigung I) der überwiegende Teil des Veresterungskatalysators mittels Wasserwäsche extraktiv abgetrennt und
2. in einer zweiten Vorreinigungsstufe (Vorreinigung II) die sauren Komponenten mit einer wässrigen Alkalilösung durch Reaktivextraktion neutralisiert und extrahiert werden und
3. optional, in einer dritten Vorreinigungsstufe (Vorreinigung III), aus dem nach der zweiten Vorreinigungsstufe verbleibenden organischen Reaktionsrestgemisch restliche Salze sowie wässrige Fremdphasenanteile mit Wasser extraktiv entfernt werden,
das dabei verbleibende organische Reaktionsrestgemisch I in eine weitere Rektifikationseinheiten umfassende Trennzone leitet und in dieser den gebildeten n-Butylester der Acrylsäure abtrennt, indem man
- das verbleibende Reaktionsrestgemisch I einer Rektifikationseinheit II zuführt und in dieser das verbliebene Reaktionsrestgemisch I in ein Acrylsäure-n-butylester und leichter als der Acrylsäure-n-butylester siedende Bestandteile enthaltendes Leichtsiederprodukt und in ein den Acrylsäure-n-butylester und schwerer als der Acrylsäure-n-butylester siedende Bestandteile umfassendes Reaktionsrestgemisch II rektifikativ auftrennt,
- das Reaktionsrestgemisch II einer Rektifikationseinheit III zuführt und in dieser den Acrylsäure-n-butylester von den schwerer als der Acrylsäure-n-butylester siedenden Bestandteilen abtrennt.

DE 198 51 983 A1 (BASF AG) beschreibt ein Verfahren zur kontinuierlichen Herstellung von Alkylestern der (Meth)acrylsäure durch Umsetzung von (Meth)acrylsäure mit 1 bis 8 C-Atome aufweisenden Alkanolen, bei dem Essigsäure enthaltende Roh-(Meth)acrylsäure als Ausgangsverbindung eingesetzt und als Nebenprodukt gebildetes Alkylacetat an geeigneter Stelle ausgeschleust wird.

DE 199 35 453 A1 (BASF AG) beschreibt ein Verfahren zur kontinuierlichen Herstellung von Acrylestern der (Meth)acrylsäure durch Umsetzung von (Meth)acrylsäure mit 4 bis 8 C-Atome aufweisenden Alkanolen in einer Veresterungszone, wonach das aus der Veresterungszone abgezogene Reaktionsgemisch zunächst einer dreistufigen Vorreinigung zugeführt und anschließend zur Abtrennung des Zielesters rektifikativ aufgearbeitet wird.

Das oben beschriebene BASF-Extraktionsverfahren ist hauptsächlich fokussiert auf Maximierung der Ausbeute und Ausschleusung der leichtsiedenden Nebenkomponente n-Butylacetat. Wenn das Verfahren auf diese Weise durchgeführt wird, gelingt es wirtschaftlich und effizient, ein Produkt mit niedrigerem Gehalt an dem Acetat zu gewinnen. In der betrieblichen Praxis zeigt sich jedoch, dass neben Butylacetat eine weitere Nebenkomponente, nämlich Di-n-butyl-ether (DBE), vermehrt gebildet wird und im Produkt akkumuliert. Die typischen Konzentrationen an dem Dibutylether im Reinprodukt, betragen z.B. 700 bis 2000 ppm.

Der vorliegenden Erfindung lag die Aufgabe zugrunde, ein Verfahren zur Herstellung von n-Butylacrylsäureester durch Veresterung von n-Butanol mit Acrylsäure mit anschließender extraktiver und destillativer Aufarbeitung des Rohesters und Spaltung der Nebenprodukte "Butoxyester" und "michaeloligomere Acrylsäurebutylester", zur Verfügung zu stellen, mit dem der n-Butylacrylsäureester in hoher Reinheit, d. h. mit einem geringen Anteil an Nebenkomponenten, insbesondere mit einem niedrigen Anteil an Di-n-butyl-ether, und hoher Ausbeute erhalten werden kann.

Die Aufgabe bestand insbesondere darin, Möglichkeiten zur Veränderung des besonders in DE 198 51 983 A1 in Verbindung mit DE 199 35 453 A1 beschriebenen Prozesses zu finden, die zu einer Reduktion der Konzentration an DBE im Verfahrensprodukt n-Butylacrylat (n-BA) führen würden.

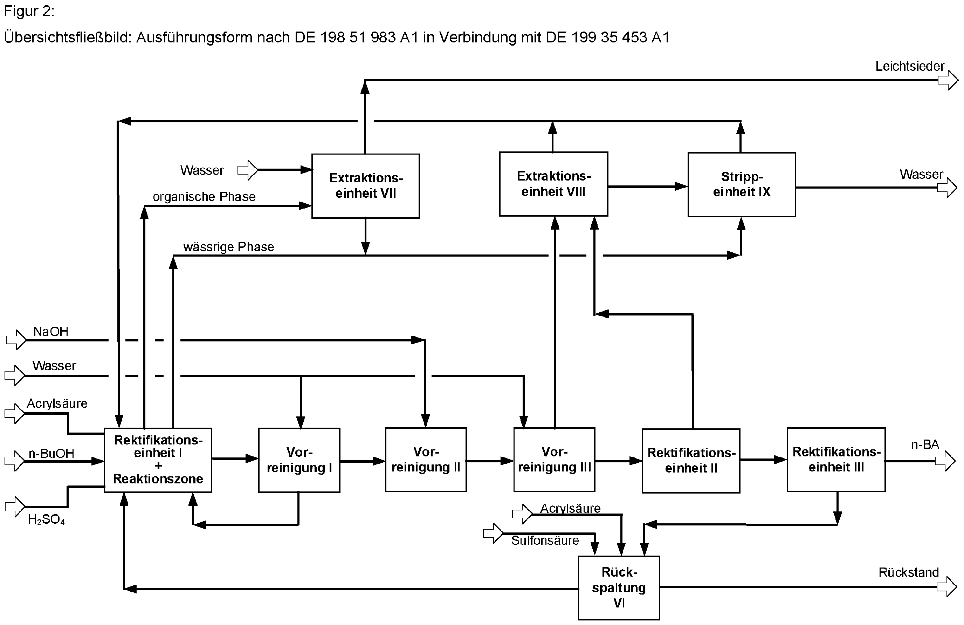

Figur 2 zeigt als Übersichtsfließbild eine Ausführungsform des Verfahrens nach DE 198 51 983 A1 in Verbindung mit DE 199 35 453 A1.

Es ist aus der Literatur bekannt, dass die Bildung des Ethers DBE über Dehydratisierung/Dimerisierung von n-Butanol an sauren Katalysatoren bei erhöhten Temperaturen erfolgt. Solche Bedingungen herrschen bei der n-BA-Synthese sowohl in dem Veresterungsteil als auch in der Rückspaltung. Erfindungsgemäß wurde zu Lösung der Aufgabe des Erarbeitens des verbesserten Verfahrens zur Synthese von n-Butylacrylat mit niedriger Konzentration an Di-n-butyl-ether erstens untersucht, in wie weit die Etherbildung in der Synthese, Rückspaltung oder in beidem erfolgt. Des Weiteren wurde erfindungsgemäß untersucht ob durch Optimierung der Reaktionsbedingungen und/oder Rückspaltbedingungen, eine günstige Beeinflussung der Etherbildungsrate möglich ist. Erfindungsgemäß wurden also zwei Möglichkeiten, die zur Verfügung stehen, erkannt:
Die Reaktionsbedingungen in der Synthese und/oder in der Rückspaltung zu optimieren.

Obwohl es aus der Literatur hervorgeht, dass ein wesentlicher Teil des betreffenden Ethers in der Rückspaltung gebildet wird, hat sich die alleinige Optimierung der Rückspaltbedingungen nicht als wirtschaftlich genug erwiesen, da durch Einstellung von milderen Reaktionsbedingungen (tiefere Temperatur, niedrigere Katalysatorkonzentration), die die n-Butanol-Dehydratisierung begünstigen, parallel die Rückspaltrate abnimmt, was mit der Zunahme an Rückstand und somit erhöhten Rohstoffverlusten resultiert.

Das im EP 765 861 B1 (BASF AG) beschriebene Verfahren zur Durchführung der Rückspaltung unter Vakuum, das in einer reduzierten Di-Alkylether-Bildung resultiert, kann zur Lösung der vorliegenden Aufgabe nicht alleinig ohne weiteres angewandt werden. Durch die Erniedrigung des Druckes, sinkt die Siedetemperatur des Gemisches, gleichzeitig aber, um die Spaltung effektiv durchzuführen, ist eine Temperatur von 180 °C im Sumpf vorteilhaft. Dies hat eine Verdampfung von Schwersiedern zur Folge. Dies erfordert wiederum einen Aufbau einer Rektifikationseinheit mit mehreren theoretischen Trennstufen, die eine Rückhaltung der Schwersieder im Sumpf ermöglichen. Unabhängig davon, erfordert diese Verfahrensvariante eine Rektifikation von Acrylsäure-haltigen Strömen, was bei den hohen Temperaturen die Polymerisation von Acrylsäure in der Rektifikationseinheit beschleunigt und zu häufigen Abstellungen führen kann.

Die Aufgabe wurde gelöst durch ein Verfahren zur kontinuierlichen Herstellung von Acrylsäure-n-butylester durch Umsetzung von Acrylsäure mit n-Butanol in an Lösungsmittel freier Phase bei erhöhter Temperatur und unter Zusatz von Säure als saurem Veresterungskatalysator, besonders bevorzugt Schwefelsäure, bei dem man die Acrylsäure, das n-Butanol und den Veresterungskatalysator einer Reaktionszone zuführt, während einer Verweilzeit in der Reaktionszone das gebildete Wasser als Bestandteil eines n-Butanol umfassenden Gemisches in einer der Reaktionszone aufgesetzten ersten Rektifikationseinheit I vom Reaktionsgemisch abtrennt, das dabei anfallende Destillat in eine n-Butanol enthaltende organische und in eine Wasser enthaltende wässrige Phase auftrennt, die organische Phase in die Rektifikationseinheit I zurückführt, die wässrige Phase gegebenenfalls, ganz oder teilweise (z.B. einen Anteil von 50 bis 100 Gew.-%, besonders 80 bis 100 Gew.-%, ganz besonders 95 bis 100 Gew.-%, insbesondere 'ganz', also 100 Gew.-%), einer Strippeinheit IX zuführt, das den Acrylsäure-n-butylester enthaltende Reaktionsgemisch, das aus der Reaktionszone abgezogen wird, einer Vorreinigung zuführt, wobei
1. in einer ersten Vorreinigungsstufe (Vorreinigung I) der überwiegende Teil des Veresterungskatalysators mittels Wasserwäsche extraktiv abgetrennt und
2. in einer zweiten Vorreinigungsstufe (Vorreinigung II) die sauren Komponenten mit einer wässrigen Alkalilösung durch Reaktivextraktion neutralisiert und extrahiert werden und
3. optional, in einer dritten Vorreinigungsstufe (Vorreinigung III), aus dem nach der zweiten Vorreinigungsstufe verbleibenden organischen Reaktionsrestgemisch restliche Salze sowie wässrige Fremdphasenanteile mit Wasser extraktiv entfernt werden,

das dabei verbleibende organische Reaktionsrestgemisch I in eine weitere Rektifikationseinheiten umfassende Trennzone leitet und in dieser den gebildeten n-Butylester der Acrylsäure abtrennt, indem man
   - das verbleibende Reaktionsrestgemisch I einer Rektifikationseinheit II zuführt und in dieser das verbliebene Reaktionsrestgemisch I in ein Acrylsäure-n-butylester und leichter als der Acrylsäure-n-butylester siedende Bestandteile enthaltendes Leichtsiederprodukt und in ein den Acrylsäure-n-butylester und schwerer als der Acrylsäure-n-butylester siedende Bestandteile umfassendes Reaktionsrestgemisch II rektifikativ auftrennt,
   - das Reaktionsrestgemisch II einer Rektifikationseinheit III zuführt und in dieser den Acrylsäure-n-butylester von den schwerer als der Acrylsäure-n-butylester siedenden Bestandteilen abtrennt,
dadurch gekennzeichnet, dass
   - die Menge an zugesetzter Säure als Katalysator in die Reaktionszone im Bereich von 51 bis 163 mmol Säure pro kg des aus der Reaktionszone abgezogenen Reaktionsgemischs beträgt,
   - man n-Butanol und Acrylsäure in einem Massenverhältnis im Bereich von n-Butanol : Acrylsäure = 1,0 bis 1,3 einsetzt,
   - man wenigstens den organischen Anteil des nicht als Rücklauf in die Rektifikationseinheit II verwendeten Leichtsiederproduktes aus der Rektifikationseinheit II zumindest teilweise (z.B. einen Anteil von 50 bis 100 Gew.-%, besonders 80 bis 100 Gew.-%, ganz besonders 95 bis 100 Gew.-%, insbesondere vollständig, also 100 Gew.-%) einer Rektifikationseinheit IV zuführt (Strom 1), in der Wasser und n-Butanol und n-Butylacetat und Di-n-butyl-ether abdestilliert werden, das Destillat (Strom 3) zumindest teilweise (z.B. einen Anteil von 20 bis 100 Gew.-%, besonders 50 bis 100 Gew.-%, ganz besonders 70 bis 100 Gew.-%, insbesondere vollständig, also 100 Gew.-%) einer n-Butanol-Extraktionseinheit VII zuführt und das Sumpfprodukt (Strom 2) entweder direkt oder über eine Acrylsäure-Extraktionseinheit VIII in die Reaktionszone rückführt.

In einer besonders vorteilhaften Ausgestaltung ist das Verfahren weiter dadurch gekennzeichnet, dass das in der Rektifikationseinheit III anfallende Sumpfprodukt einer Rückspaltung VI im Beisein einer schwefelhaltigen mineralischen Säure, phosphorhaltigen mineralische Säure oder organischen Sulfonsäure, oder einer Mischung daraus, besonders aromatischen Sulfonsäure, ganz besonders Dodecylbenzolsulfonsäure (CAS-Nummer: 27176-87-0) oder Maranil^{®} DBS/LC (CAS-Nummer: 85536-14-7), bei erhöhter Temperatur unterworfen wird und die dabei gasförmig entweichenden Rückspaltprodukte zunächst kondensiert und dann einer Rektifikationseinheit V zuführt (Strom 4), in der Wasser, n-Butanol und Di-n-butyl-ether abdestilliert werden, und aus der das Sumpfprodukt in die Reaktionszone rückgeführt wird (Strom 5).

Die Konzentration der mineralischen Säure oder Sulfonsäure beträgt jeweils bevorzugt im Bereich von 1 bis 30 Gew.-%, weiter bevorzugt 5 bis 25 Gew.-%, besonders bevorzugt 10 bis 20 Gew.-%, weiter besonders bevorzugt 12 bis 18 Gew.-%, z.B. 15 Gew.-%, jeweils bezogen auf das Reaktionsgemisch der Rückspaltung.

Weiter besonders wird das Destillat der Rektifikationseinheit V zumindest teilweise (z.B. ein Anteil von 20 bis 100 Gew.-%, besonders 50 bis 100 Gew.-%, ganz besonders 70 bis 100 Gew.-%, insbesondere vollständig, also 100 Gew.-%) der n-Butanol-Extraktionseinheit VII zugeführt (Strom 6). In der n-Butanol-Extraktionseinheit VII wird n-Butanol mit Wasser extrahiert. Der Sumpf der Rektifikationseinheit V enthält Acrylsäure und wird deshalb bevorzugt zur Veresterung in die Reaktionszone zurückgeführt.

Bei der Säure als Veresterungskatalysator handelt es sich bevorzugt um eine schwefelhaltige mineralische Säure, phosphorhaltige mineralische Säure, organische Sulfonsäure, oder einer Mischung daraus. Bei der organischen Sulfonsäure kann es sich um eine aromatische Sulfonsäure (besonders z.B. p-Toluolsulfonsäure, Benzolsulfonsäure, Dodecylbenzolsulfonsäure (CAS-Nummer: 27176-87-0), Maranil^{®} DBS/LC (CAS-Nummer: 85536-14-7)), aliphatische Sulfonsäure (besonders z.B. Methansulfonsäure) oder einer Mischung daraus handeln. Ganz besonders bevorzugt handelt es sich bei der Säure um n-Butyl-schwefelsäure, weiter besonders Schwefelsäure.

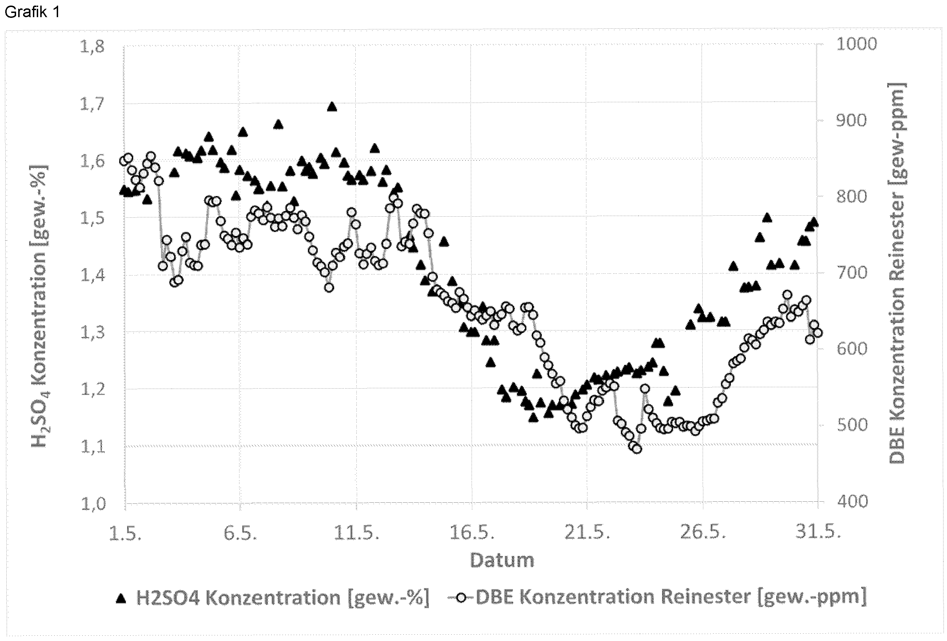

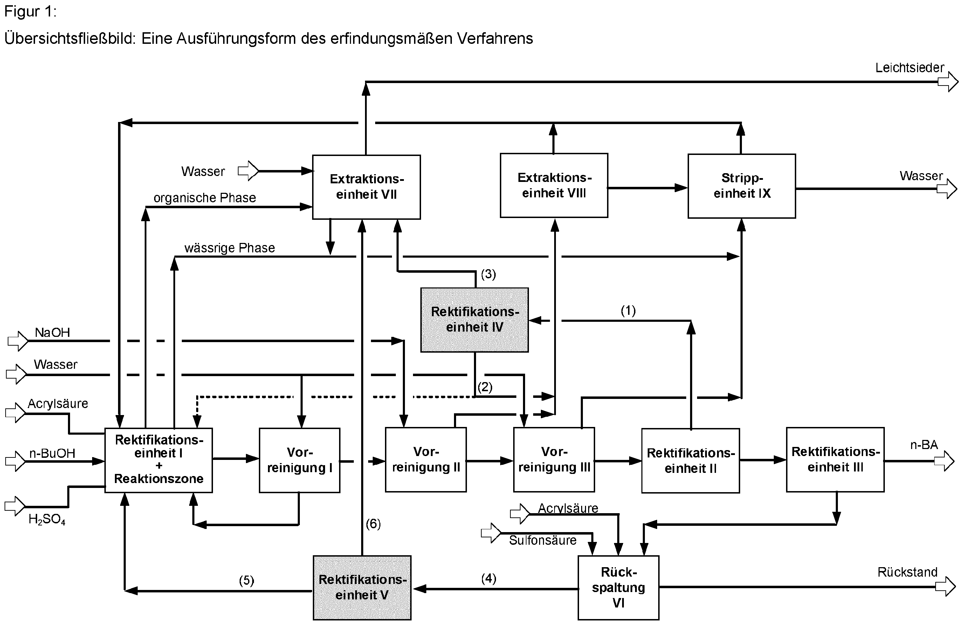

Figur 1 zeigt als Übersichtsfließbild eine Ausführungsform des erfindungsgemäßen Verfahrens.

Das erfindungsgemäße Verfahren liefert gegenüber dem besonders in DE 198 51 983 A1 in Verbindung mit DE 199 35 453 A1 beschriebenen Verfahren das n-BA mit einer wesentlich reduzierten Konzentration an DBE im Reinester. Die Raumzeitausbeute ist dabei vergleichbar gut oder besser. Gleichzeitig erniedrigt sich im Besonderen die Rückstandsmenge, was zu besseren Einsatzzahlen und Minimierung des Abfalls führt.

Weitere besonders vorteilhafte Ausgestaltungen des erfindungsgenäßen Verfahrens sind wie folgt:
Die Umsetzung von Acrylsäure mit n-Butanol wird bevorzugt bei einer Temperatur im Bereich von 70 bis 160 °C, besonders 80 bis 130 °C, weiter besonders 110 bis 125 °C, und einem Absolutdruck in Bereich von 300 bis 1500 mbar, besonders 400 bis 700 mbar, weiter besonders 500 bis 600 mbar, z.B. 550 mbar, durchgeführt.

Die Menge an zugesetzter Säure als Katalysator in die Reaktionszone beträgt bevorzugt im Bereich von 92 bis 153 mmol, besonders im Bereich von 102 bis 143 mmol, weiter besonders im Bereich von 112 bis 133 mmol, jeweils pro kg des aus der Reaktionszone abgezogenen Reaktionsgemischs.

Im Verfahren setzt man n-Butanol und Acrylsäure bevorzugt in einem Massenverhältnis im Bereich von n-Butanol : Acrylsäure = 1,05 bis 1,2, besonders im Bereich von n-Butanol : Acrylsäure = 1,1 bis 1,15, weiter besonders im Bereich von n-Butanol : Acrylsäure = 1,11 bis 1,14, z.B. 1,13, ein.

Die Rückspaltung VI des in der Rektifikationseinheit III anfallenden Sumpfprodukts wird bevorzugt bei einer Temperatur im Bereich von 120 bis 200 °C, besonders 130 bis 190 °C, weiter besonders 150 bis 180 °C, und einem Absolutdruck im Bereich von 400 bis 1500 mbar, besonders 600 bis 1200 mbar, weiter besonders 900 bis 1100 mbar, durchgeführt.

Vorteilhaft wird im Besonderen in der Acrylsäure-Extraktionseinheit VIII das Sumpfprodukt der Rektifikationseinheit IV als Extraktionsmittel verwendet.

Die Extraktionskolonne der Einheit VIII ist vorteilhaft folgendermaßen aufgebaut:
Am Kopf der Kolonne: Wasser als Extraktionsmittel um Reste an Acrylsäure und Alkalisalzen der Katalysatorsäure (besonders z.B. Natriumsulfat im Fall von Schwefelsäure als Katalysator oder Natriumsalz der n-Butyl-schwefelsäure im Fall von n-Butyl-schwefelsäure als Katalysator) noch "auszuwaschen". Im oberen Teil der Kolonne wird die zu reinigende Flüssigkeit aufgegeben (enthaltend z.B. Natriumsalz von Acrylsäure aus Vorreinigung II und ggf. III). Im unteren Teil der Kolonne wird eine Mischung aus n-Butanol und n-Butylacrylat als Extraktionsmittel aufgegeben. Die Vorreinigung III des erfindungsgemäßen Verfahrens ist optional, wenn sie nicht vorhanden ist, was weniger bevorzugt ist, dann entfällt der Strom von Vorreinigung III zur Strippeinheit IX.

Die Verweilzeit der Edukte in der Reaktionszone beträgt bevorzugt im Bereich von 0,25 bis 5 Stunden, besonders im Bereich von 0,75 bis 4 Stunden, weiter besonders im Bereich von 1 bis 3,75 Stunden, z.B. im Bereich von 1,25 bis 3,5 Stunden.

Der Restgehalt an Acrylsäure im Reaktionsgemisch, das aus der Reaktionszone abgezogen wird, beträgt bevorzugt ≤ 5,0 Gew.-%, besonders ≤ 2,0 Gew.-%, weiter besonders ≤ 1,8 Gew.-%, ganz besonders im Bereich von 1,05 bis 1,5 Gew.-%, z.B. im Bereich von 1,2 bis 1,3 Gew.-%.

Die Rückspaltung VI des in der Rektifikationseinheit III anfallenden Sumpfprodukts wird bevorzugt in einem Rührkessel oder einem Behälter mit außenliegendem Wärmeübertrager, jeweils in einer kontinuierlichen, diskontinuierlichen oder halbkontinuierlichen Fahrweise, durchgeführt.

Besonders wird die Rückspaltung VI des in der Rektifikationseinheit III anfallenden Sumpfprodukts unter Zugabe von Acrylsäure und/oder michaeloligomerer Acrylsäure in einer Menge (Gesamtmenge an diesen Säuren) im Bereich von 1 bis 20 Gew.-%, besonders im Bereich von 5 bis 15 Gew.-%, weiter besonders im Bereich von 10 bis 14 Gew.-%, jeweils bezogen auf die Menge des in der Rektifikationseinheit III anfallenden Sumpfprodukts, durchgeführt. Diese Maßnahme reduziert zusätzlich oder verhindert sogar völlig die Di-n-butyl-ether-Bildung.

Bevorzugt werden in der Strippeinheit IX organische Komponenten mit Wasserdampf oder durch indirekte Beheizung (Verdampfer besonders im Sumpf der Kolonne) vom Wasser getrennt und über den Kopf der Strippeinheit zur Rektifikationseinheit I zurückgeführt.

Einen Teil der organischen Phase, die nach Phasentrennung des Destillats der Rektifikationseinheit I anfällt, leitet man bevorzugt in eine n-Butanol-Extraktionseinheit VII und die dort anfallende wässrige Phase in eine anschließende Strippeinheit IX, wobei die dort zurückgewonnene organische Phase in die Rektifikationseinheit I zurückgeführt wird.

Bevorzugt wird ein überwiegender Anteil der organischen Phase, die nach Phasentrennung des Destillats der Rektifikationseinheit I anfällt, z.B. 70 bis 100 Gew.-%, besonders 90 bis 100 Gew.-%, ganz besonders 95 bis 99 Gew.-%, als Rücklauf in die Rektifikationseinheit I zurückgeführt. Der verbleibende Anteil des organischen Rücklaufes kann zwecks Ausschleusung von n-Butylacetat, aber auch DBE, als Leichtsieder-Abfall, an dieser Stelle aus dem System entzogen werden. Da dieser Strom noch n-Butanol enthält, ist es wirtschaftlich, aus diesem Strom n-Butanol zurückzugewinnen, was in der n-Butanol-Extraktionseinheit VII geschieht. Somit geht der Leichtsiederstrom, also Raffinat der Extraktionseinheit VII, zur Entsorgung, z.B. Verbrennung, und der wässrige Extrakt, beladen mit n-Butanol, geht in die Strippeinheit IX, wo aus dem wässrigen Strom n-Butanol ausgestrippt wird und bevorzugt zurück in die Synthese gefahren wird.

Das Veresterungswasser, also die wässrige Phase des Destillats aus der Rektifikationseinheit I, ist in der Regel mit organischen Verbindungen gesättigt und kann bevorzugt, ganz oder teilweise, ebenfalls in der Strippeinheit IX zusammen mit wässrigem Extrakt aus der n-Butanol-Extraktionseinheit VII von organischen Verbindungen befreit werden.

Durch die erfindungsgemäße Installation der Kolonnen in den Rektifikationseinheiten IV und bevorzugt V, erfolgt eine Ausschleusung von n-Butylacetat und DBE analog via Extraktionseinheit VII und Strippeinheit IX über die organischen Phasen der Kolonnenkopfprodukte der Rektifikationseinheit IV und ggf. Rektifikationseinheit V.

Dadurch gibt es bis zu drei Leichtsiederströme über die n-Butylacetat und DBE ausgeschleust werden können. In der Regel ist die Konzentration an DBE in dem Leichtsiederstrom aus der Rektifikationseinheit I am geringsten, so dass auf die Ausschleusung von DBE über diesem Strom alternativ auch verzichtet werden kann.

Mit dem erfindungsgemäßen Verfahren lässt sich Acrylsäure-n-butylester in einer Reinheit von besonders ≥ 99,5 Gew.-%, weiter besonders 99,6 bis 99,9 Gew.-%, z.B. 99,7 bis 99,8 Gew.-%, und mit einem Gehalt an Di-n-butyl-ether (DBE) von besonders ≤ 400 Gew.-ppm, weiter besonders ≤ 300 Gew.-ppm, z.B. 100 bis 290 Gew.-ppm, herstellen.

Der Gehalt an n-Butylacetat im hergestellten Acrylsäure-n-butylester beträgt besonders
≤ 50 Gew.-ppm, ganz besonders ≤ 30 Gew.-ppm.

In der besonders vorteilhaften Ausgestaltung des erfindungsgemäßen Verfahrens mit einer Rückspaltung VI und einer Rektifikationseinheit V, gemäß Anspruch 2, lässt sich Acrylsäure-n-butylester in einer Reinheit von besonders ≥ 99,5 Gew.-%, weiter besonders 99,6 bis 99,9 Gew.-%, z.B. 99,7 bis 99,8 Gew.-%, und mit einem Gehalt an Di-n-butyl-ether (DBE) von besonders ≤ 200 Gew.-ppm, ganz besonders ≤ 175 Gew.-ppm, z.B. 50 bis 180 Gew.-ppm, herstellen.

Im Folgenden werden weitere mögliche Ausgestaltungen des Verfahrens aufgeführt:
Als Edukt für die Veresterung eignet sich insbesondere eine Acrylsäure, die auf dem Weg der katalytischen Gasphasenoxidation von C3-Vorläufern, wie Propan, Propen, Acrolein, mit molekularem Sauerstoff erzeugt wurde. Die auf diesem Weg gewonnene, sogenannte Roh-Acrylsäure kann vorzugsweise unmittelbar der Veresterung zugeführt werden, es kann jedoch als Säureedukt ebenso jede höher gereinigte Acrylsäure verwendet werden. Roh-Acrylsäure enthält als Verunreinigungen insbesondere bis zu 5 Gew.-% Essigsäure, daneben häufig noch bis zu 1 Gew.-% Maleinsäure und/oder deren Anhydrid sowie niedermolekulare Aldehyde, häufig bis zu 0,5 Gew.-%, sowie bis zu 0,5 Gew.-% sonstige Bestandteile, beispielsweise Propionsäure, Diacrylsäure sowie Polymerisationsinhibitoren, beispielsweise Phenothiazin. Der Acrylsäuregehalt der vorzugsweise verwendbaren Roh-Acrylsäure beträgt häufig mindestens 95 Gew.-%, vielfach mindestens 97 oder mindestens 98 oder mindestens 99 Gew.-%. Auch das Alkanoledukt n-Butanol wird bevorzugt in einer Reinheit von mindestens 95 Gew.-%, vielfach mindestens 97 oder mindestens 98 oder mindestens 99 Gew.-% eingesetzt.

Als Veresterungskatalysator kommt, wie oben beschrieben, eine Säure zum Einsatz. Besonders bevorzugt ist Schwefelsäure. Die Schwefelsäure reagiert unter den gegebenen Reaktionsbedingungen, zumindest teilweise, mit n-Butanol zu n-Butyl-schwefelsäure (= Schwefelsäure-monon-butylester).

Die Veresterung erfolgt analog der in DE 198 51 983 A1 (BASF AG) beschriebenen Weise in einer Reaktionszone, die aus einem oder mehreren Reaktionsbereichen bestehen kann. Bei einer Ausgestaltung des Verfahrens mit mehreren Reaktionsbereichen ist es vorteilhaft, diese zu kaskadieren. Der flüssige Austragsstrom eines Reaktionsbereiches bildet dabei zweckmäßigerweise den Zulauf des nachfolgenden Reaktionsbereiches. Dies kann in einfacher Weise mit Hilfe eines Überlaufs oder aber auch mit Pumpen geschehen. Für den Fall, dass es sich bei den einzelnen Reaktionsbereichen um voneinander getrennte Apparate handelt, ist deren Anzahl unter Berücksichtigung der Investitionskosten zweckmäßig ≥ 2 und ≤ 4. Wird mehr als ein Reaktionsbereich innerhalb ein und desselben Reaktors geschaffen (z.B. durch den Einsatz von Trennblechen), so kann die Anzahl der Reaktionsbereiche auch größer als 4 sein. Im Fall von mehreren Reaktionsbereichen können die Brüden der einzelnen Reaktionsbereiche einer gemeinsamen Rektifikationseinheit (I), z.B. einer gemeinsamen Rektifikationskolonne, zugeführt werden, deren flüssiger Ablauf zweckmäßigerweise dem ersten Reaktionsbereich entsprechend zugeführt wird. Erfindungsgemäß kann es jedoch sinnvoll sein, auf mehrere Reaktionsbereiche, gegebenenfalls auf alle, je eine Rektifikationseinheit aufzusetzen und deren flüssigen Rücklauf entsprechend in einen oder mehrere Reaktionsbereiche, zweckmäßigerweise in diejenigen, auf die die Rektifikationseinheiten aufgesetzt sind, zurückzuführen. Häufig wird auf den ersten Reaktionsbereich keine Rektifikationseinheit aufgesetzt.

Der Begriff Rektifikationseinheit ist hier wie auch im Folgenden als allgemeine Bezeichnung für Vorrichtungen zu verstehen, in denen durch Wärmezufuhr erzeugte Dämpfe aufsteigen und in Kontakt mit abströmender flüssiger Phase stehen. In der Regel handelt es sich hierbei um Rektifikationskolonnen, in denen Einbauten für den intensiven Kontakt zwischen Flüssigkeit und Dampf enthalten sind. Bevorzugt werden als Einbauten in sämtliche Rektifikationseinheiten Dual-Flow-Böden und/oder strukturierte Packungen eingesetzt.

Wie bereits oben beschrieben, kann die Veresterung in der Reaktionszone bei vermindertem Druck (d. h. < 1 bar) betrieben werden, was i.d.R. die rektifikative Abtrennung des Reaktionswassers über die Rektifikationseinheit I erleichtert, oder sie kann auch drucklos (d. h. bei 1 bar) oder auch bei Überdruck durchgeführt werden. Normalerweise ist die Reaktionszone mit der aufgesetzten Rektifikationseinheit I von den übrigen Rektifikationseinheiten sowohl räumlich als auch regeltechnisch getrennt. Die Bedingungen im Reaktionsbereich und in den zur Abtrennung des Zielesters verwandten Rektifikationseinheiten sind daher sehr flexibel einstellbar.

Die Temperatur des Reaktionsgemisches in den Reaktionsbereichen entspricht normalerweise dem eingestellten Druck, sowie der im Reaktionsbereich vorliegenden Zusammensetzung des Reaktionsgemisches. D. h., im Fall einer Kaskadierung (im Fall von mehreren Reaktionsbereichen) nimmt die Reaktionstemperatur in der Regel längs der Kaskade zu (der Reaktionsdruck wird üblicherweise längs der Kaskade konstant gehalten).

Die Gesamtverweilzeit der Reaktanden in der Reaktionszone wird vorzugsweise so eingestellt, dass ein Umsatz von ≥ 90 Gew.-%, bevorzugt ein Umsatz im Bereich von 94 bis 99 Gew,-%, weiter bevorzugt im Bereich von 95 bis 98,5 Gew.-%, z.B. 96 bis 98 Gew.-%, jeweils bezogen auf Acrylsäure, erreicht wird. In aufeinanderfolgenden Reaktionsbereichen nimmt die Verweilzeit der Reaktanden normalerweise ab.

Das in der Rektifikationseinheit I, im Normalfall am Kopf einer Rektifikationskolonne, anfallende Destillat wird durch das Abkühlen beim Kondensieren (alternativ kommt auch eine rektifikative Phasentrennung in Betracht), in eine organische und in eine wässrige Phase aufgetrennt. Die organische Phase besteht überwiegend aus organischen Bestandteilen (hauptsächlich n-Butanol, n-Butylacetat, n-Butylacrylat und Di-n-butyl-ether), während die wässrige Phase überwiegend aus Veresterungswasser besteht.

Die in die Rektifikationseinheit I rückgeführte Menge an organischer Phase wird in der Regel so bemessen, dass das Rücklaufverhältnis (das Verhältnis von rückgeführter Menge zu entnommener Menge) 5 bis 40, vorzugsweise 10 bis 30 beträgt. Insgesamt wird die Trennleistung in der Rektifikationseinheit I häufig so gewählt, dass der n-Butylacetatgehalt der entnommenen organischen Phase wenigstens 5 Gew.-%, in der Regel wenigstens 10 Gew.-%, vielfach wenigstens 20 Gew.-% beträgt.

Die Rektifikationseinheit I kann aus einer oder mehreren Rektifikationskolonnen bekannter Bauart, insbesondere mit Dual-Flow-Böden oder strukturierten Packungen oder Siebböden bestehen. Normalerweise wird sie durch zugehörige Kondensatoren und Trenngefäße ergänzt. Die Reaktionsbereiche können z.B. aus Reaktoren mit Natur- oder Zwangsumlaufverdampfer bestehen. D. h., die Durchmischung des Reaktionsgemischs kann durch Rühren, Umpumpen und/ oder Naturumlauf erfolgen. Die Wärmezufuhr erfolgt in an sich bekannter Weise, z.B. durch Doppelwandbeheizung oder außen- und/oder innenliegende Wärmetauscher. Zur Stabilisierung der Rektifikationseinheit I gegen unerwünschte Bildung von radikalisch initiiertem Polymerisat wird, zweckmäßigerweise auf deren Kopf, eine Lösung eines Polymerisationsinhibitors aufgebracht. Als Lösungsmittel eignet sich diesbezüglich z. B. das Zielprodukt n-Butylacrylat oder die in die Rektifikationseinheit I rückzuführende organische Phase des in der Rektifikationseinheit IV anfallenden Sumpfes. Vorzugsweise wird als Polymerisationsinhibitor Phenothiazin eingesetzt. Die Kondensatoren (z. B. Platten- oder Rohrbündelkondensatoren), in welchen die in der Rektifikationseinheit I aufsteigenden Brüden zum Gewinn des in der Rektifikationseinheit I abzutrennenden Destillates kondensiert werden, werden zweckmäßigerweise ebenfalls mittels an sich bekannten Polymerisationsinhibitoren stabilisiert. Mit Vorteil wird dazu eine wässrige Lösung (ca. 0,1-1 Gew.-%ig) wenigstens eines (in der Regel wenigstens eine Wasserlöslichkeit von 1 Gew.-% (25 °C, 1 bar) aufweisenden) Inhibitors auf den Kondensator aufgebracht und/oder dem Kondensat zugegeben. Als solche wasserlöslichen Inhibitoren kommen z.B. Hydrochinon, p-Nitrosophenol, Phenylendiamine wie Kerobit PD (N,N'-Diisobutyl-p-phenylendiamin), p-Nitrosodiethylanilin, 2,2,6,6-Tetramethylpiperidin-N-oxyl, 4-Hydroxy- 2,2,6,6-tetramethylpiperidin-N-oxyl oder Gemische aus den vorgenannten Vertretern in Betracht. Bevorzugt wird eine wässrige Lösung die 4-Hydroxy-2,2,6,6-tetramethylpiperidin-N-oxyl enthält (vorzugsweise als alleinigen Inhibitor) verwendet. Bevor der in der Regel im Wesentlichen aus Zielester, nicht umgesetzter Acrylsäure, leichter als der Zielester siedenden Nebenprodukten wie n-Butylacetat oder Di-n-butyl-ether, Veresterungskatalysator, durch Michael Addition gebildeten höher siedenden Oxyestern und Polymerisationsinhibitor bestehende Austrag aus der Reaktionszone der weitere Rektifikationszonen umfassenden Trennzone zugeführt wird, wird zunächst der überwiegende Teil des Veresterungskatalysators aus dem Reaktoraustrag in einer ersten Vorreinigungsstufe (I) abgetrennt. Dies kann in Einzelheiten auf unterschiedliche Art und Weise erfolgen.

Im Fall von Mineralsäuren, z. B. Schwefelsäure, und/oder organischen Sulfonsäuren kann diese Abtrennung in besonders einfacher, an sich bekannter, Weise z.B. durch Waschen des Veresterungsaustrags mit Wasser erfolgen. Die dabei anfallende, den Veresterungskatalysator (z.B. n-Butyl-schwefelsäure) und nicht umgesetzte Acrylsäure sowie gegebenenfalls Prozesspolymerisationsinhibitor enthaltende, wässrige Phase kann in zweckmäßiger Weise unmittelbar in die Reaktionszone rückgeführt, entsorgt und/oder mit der im nächsten Abschnitt erwähnten alkalischen wässrigen Phase zum Zweck der Rückextraktion vereinigt werden. Durch eine Kreisführung wird der Bedarf an frischem Veresterungskatalysator reduziert. Dabei werden auch schwersiedende Komponenten, die in Wasser löslich sind, im Kreis gefahren, insbesondere Maleinsäureanhydrid; um eine Apparateverschmutzung, insbesondere der Wärmetauscher, als Folge der Schwersiedeanreicherung zu reduzieren oder zu vermeiden, wird daher vorteilhaft ein Teilstrom der erwähnten wässrigen Phase, bevorzugt etwa 5 Gew.-%, besonders bevorzugt etwa 10 Gew.-% der wässrigen Phase, aus dem Kreisstrom ausgeschleust. Der Verschmutzungsgrad der Wärmetauscher, gemessen an der maximalen Betriebsdauer nach der der Betrieb aufgrund von unzureichendem Wärmeeintrag abgebrochen werden musste, kann durch Ausschleusung eines Teilstroms von ca. 10 Gew.-% der wässrigen Phase gegenüber der vollständigen Rückführung derselben in die Veresterung um den Faktor 4 reduziert werden.

Die organische Phase aus der ersten Vorreinigungsstufe, die neben dem Zielester insbesondere noch n-Butanol, Acrylsäure sowie Katalysator (und/oder Folgeprodukte davon) enthält, wird einer zweiten Vorreinigungsstufe (II) zugeführt, worin die stark sauren Komponenten mittels einer wässrigen Alkalilösung durch Reaktivextraktionen neutralisiert und extrahiert werden. Die als Extraktionsmittel eingesetzte Alkalilösung ist vorzugsweise eine wässrige Natrium- und/oder Kaliumhydroxidlösung, bevorzugt in einer Konzentration von 1 bis 50 Gew.-%, besonders bevorzugt von 6 bis 15 Gew.-%. Ganz besonders vorteilhaft ist die Verwendung einer 6 Gew.-%igen Natriumhydroxidlösung. Die Menge der zugegebenen wässrigen Natriumhydroxidlösung wird durch pH-Messung überwacht, sie richtet sich nach dem Äquivalenzpunkt des Natriumacrylats.

Das so gewonnene Gemisch läuft in einen Phasenscheider ab, wo sich die wässrige Phase absetzt, die etwa 10 Gew.-% Alkaliacrylat (besonders Natriumacrylat) enthält. Durch Ansäuern, insbesondere mit Schwefelsäure, besonders bevorzugt mit dem in der ersten Vorreinigungsstufe ausgeschleusten wässrigen Teilstrom, wird Acrylsäure freigesetzt und in einer Gegenstromkolonne mit im wesentlichen Alkanol, beispielsweise n-Butanol extrahiert. Das resultierende, Acrylsäure enthaltende Extrakt wird in die Reaktionszone in die Veresterung zurückgeführt. Das Abwasser wird, nach Freistrippung des n-Butanols zur Kläranlage geführt.

Die leichtere, organische Phase, die noch etwa 0,5 bis 15 Gew.-%, insbesondere 1 bis 10 Gew.-% Fremdphasenanteile, d. h. wässrige Phasenanteile enthält, wird optional einer dritten Vorreinigungsstufe (III) zugeführt, worin restliche Salze, insbesondere Alkalisalze (besonders Natriumsalze) der Acrylsäure und des Katalysators sowie wässrige Fremdphasenanteile mit Wasser extraktiv entfernt werden.

Dazu wird die aus der zweiten Vorreinigungsstufe abgezogene organische Phase mit Wasser, bevorzugt mit n-butanolgesättigtem Wasser, durchmischt und in einem weiteren Phasenscheider in eine leichtere organische und eine schwerere wässrige Phase aufgetrennt.

Das aus der zweiten Vorreinigungsstufe verbleibende organische Reaktionsrestgemisch wird mit Wasser bevorzugt in einem Rührkessel vermischt, der grundsätzlich mit einem Rührertyp beliebiger Bauart ausgestattet sein kann. Besonders bevorzugt ist der Rührkessel mit einem ein- oder mehrstufigen Impellerrührer bestückt.

Vorteilhaft wird in der dritten Vorreinigungsstufe zum organischen Reaktionsrestgemisch eine Wassermenge im Bereich von 0,05 bis 1, bevorzugt von 0,1 bis 0,5, besonders bevorzugt von 0,2 Gewichtsteilen, auf 1 Gewichtsteil organische Phase zugegeben.

In bevorzugter Weise kann, das in der dritten Vorreinigungsstufe zugegebene Wasser vorab eingesäuert werden, insbesondere in der Weise, dass die wässrige Phase nach der Extraktion einen pH-Wert von 6 aufweist, bevorzugt einen pH-Wert im Bereich von 3 bis 6.

Der Rührkessel ist bevorzugt in der Weise ausgelegt, dass der Leistungseintrag über den Rührer im Bereich von 0,1 bis 2 kW/m³, bevorzugt im Bereich von 0,5 bis 1 kW/m³, besonders bevorzugt bei 0,8 kW/m³, liegt.

Bevorzugt wird eine Verweilzeit im Rührkessel im Bereich von 0,5 bis 60 Min., insbesondere von 10 bis 30 Min., weiter bevorzugt von 20 Min., eingestellt. Das insbesondere im Rührkessel erhaltene Gemisch wird in einen weiteren Phasenscheider geleitet, wo es sich in eine schwerere wässrige und eine leichtere organische Phase auftrennt. Die wässrige Phase kann vorteilhaft zur Verdünnung der in der zweiten Vorreinigungsstufe einzusetzenden Alkalilösung eingesetzt werden. In der wässrigen Phase noch enthaltenes Rest-Alkaliacrylat (z.B. Natriumacrylat) kann auf dem Extraktionsweg (Ansäuern und anschließend Gegenstromextraktion mit n-Butanol) zurückgewonnen und in die Veresterung rückgeführt werden.

Die leichtere organische Phase wird anschließend der rektifikativen Aufarbeitung zur Gewinnung des Zielesters zugeführt.

Zur Durchführung der ersten und/oder dritten Vorreinigungsstufe wird erfindungsgemäß vorzugsweise eine Vorrichtung in Form einer Mixer-Settler-Anordnung eingesetzt, wobei der Mixer als mit losen Füllkörpern oder Einbauten gefülltes Rohr ausgebildet ist. Dadurch wird eine besonders gute Phasendurchmischung gewährleistet.

Das auf diese Weise verbleibende Reaktionsrestgemisch I wird (üblicherweise in der oberen Hälfte) einer Rektifikationseinheit II zugeführt und in dieser in ein hauptsächlich aus dem Zielester und höher als der Zielester siedenden Bestandteile (z. B. die Oxyester) umfassendes Reaktionsrestgemisch II sowie ein Gemisch aus leichter als der Zielester siedenden Bestandteilen (Restmengen an Wasser, n-Butanol, n-Butylacectat, Di-n-butyl-ether) und Zielester umfassendes Leichtsiederprodukt aufgetrennt.

Die Rektifikationseinheit II kann z.B. aus einem Wärmetauscher herkömmlicher Bauart, z.B. einem außenanliegenden Rohrbündelwärmetauscher sowie einer Rektifikationskolonne mit herkömmlichen Einbauten, z.B. Dualflow-Böden, Siebböden, Glockenböden, Schüttungen oder gerichteten Packungen bestehen, und durch einen Kondensator für die Brüden des Leichtsiederproduktes, z.B. einem Platten- oder Rohrbündelkondensator ergänzt sein. An diesen schließt sich in der Regel noch ein Phasentrenngefäß an, in welchem das kondensierte Leichtsiederprodukt in eine Restwasserphase und eine organische Phase aufgetrennt wird (alternativ kann die Phasentrennung auch rektifikativ bewirkt werden). Vorzugsweise wird die Restwasserphase abgetrennt und der bereits mehrfach erwähnten Alkanolstrippkolonne zugeführt. Ein Teil der organischen Phase des Leichtsiederproduktes wird als Rücklauf (das Rücklaufverhältnis wird in der Regel 3 bis 20, häufig 5 bis 10 betragen) in die Rektifikationseinheit II rückgeführt und der nicht als Rücklauf verwendete Teil der organischen Phase des Leichtsiederproduktes wird in erfindungsgemäßer Weise (s.o.) in die Reaktionszone rückgeführt. Einen Teil des in der Rektifikationskolonne IV anfallenden Sumpfes kann man zur Herstellung einer Phenothiazinlösung verwenden und diese über den Kopf der Rektifikationseinheit I, die Rektifikationseinheit I damit stabilisierend, in die Reaktionszone rückführen. Zur Stabilisierung der Rektifikationseinheit II kann von vorgenannter Phenothiazinlösung auch als Rücklauf in die Rektifikationseinheit II, vorzugsweise in deren Kopf, rückgeführt werden. Der zur Rektifikationseinheit II gehörige Kondensator wird in der Regel ebenfalls polymerisationsstabilisiert betrieben. Als Stabilisatorlösung wird dazu vorzugsweise eine Lösung eines oder mehrerer der nachfolgenden Polymerisationsinhibitoren ausgewählt aus der Gruppe umfassend Thiodiphenylamine (z. B. Phenothiazin), Nitrosoverbindungen (z.B. p-Nitrosophenol), Nitroxylverbindungen (z.B. 2,2,6,6-Tetramethylpiperidin-N-oxyl) und Phenylendiamine (z.B. N,N'-Diisobutyl-p-phenyldiamin = Kerobit BPD) auf die Kondensatoroberfläche aufgebracht und/oder dem Kondensat zugegeben. Als Lösungsmittel wird zweckmäßigerweise Zielester oder organische Phase des Leichtsiederproduktes verwendet.

Das Reaktionsrestgemisch II wird der Rektifikationseinheit II in der Regel als Sumpfprodukt entnommen und einer weiteren Rektifikationseinheit III (üblicherweise in die untere Hälfte einer herkömmlichen Rektifikationskolonne) zugeführt. In dieser kann der Zielester in hoher Reinheit und im Wesentlichen frei von Alkylacetaten als Kopfprodukt abgetrennt werden. Der zugehörige Kondensator wird vorzugsweise ebenfalls polymerisationsstabilisiert betrieben. Als Polymerisationsinhibitor eignet sich an dieser Stelle mit Vorteil eine Lösung von Hydrochinonmonoethylether (MEHQ) in Zielester. Auf diese Weise kann das Zielprodukt in mittels 10-20 Gew.-ppm MEHQ als Lagerstabilisator enthaltender Form gewonnen werden. Ein Teil des so stabilisierten Produktes wird als Rücklauf (häufig bei einem Rücklaufverhältnis von 0,1 bis 5 bzw. 0,3 bis 1) in die Rektifikationseinheit III rückgeführt. Im Übrigen ist es zweckmäßig, die Rektifikationseinheit III zusätzlich mittels einer ca. 0,01 bis 2 Gew.-%igen Lösung von MEHQ in Zielester zu stabilisieren. Im Fall einer als Rektifikationseinheit III verwendeten Rektifikationskolonne wird man den (in der Regel mit 10 bis 20 Gew.-ppm) MEHQ-stabilisierten Rücklauf in der Regel am Kopf der Kolonne rückführen und die zusätzliche 0,01 bis 2 Gew.-%ige MEHQ-Lösung in Zielester auf einen Kolonnenboden der oberen Kolonnenhälfte oberhalb des Zulaufs des Reaktionsrestgemisches II aufgeben.

Das bereits erwähnte n-Butanolstrippen der im vorstehend beschriebenen Verfahren an verschiedenen Stellen anfallenden Abwässer kann beispielsweise in einem beheizbaren Rührreaktor mit einer aufgesetzten Kolonne oder nach dem Gegenstromprinzip in einer Strippkolonne durchgeführt werden. Der Energieeintrag kann dabei in an sich bekannter Weise erfolgen (z.B. Doppelwand-Rohrschlangenheizung, Umlauferhitzer etc.). Dabei kann das n-butanolhaltige Abwasser z.B. am Kopf der Kolonne eingespeist und im Gegenstrom mit Wasserdampf (0,1 bis 10 t/m³, Temp.: z.B. 144 °C, Druck: z.B. 4 bar) gestrippt werden. Das Kondensat zerfällt in eine Wasser- und in eine n-Butanolphase, wovon die letztere entweder direkt in die Reaktionszone rückgeführt oder bei der Rückextraktion von Acrylsäure aus den Waschwassern, z.B. der Alkaliwaschung, mit verwendet werden kann. Das verstehend beschriebene Alkanolstrippen kann gegebenenfalls auch durch eine einfache rektifikative Abtrennung ersetzt werden.

Die Betriebsbedingungen der Rektifikationseinheiten II und III sind in typischer Weise z.B.: Rektifikationseinheit II:
- Kopftemperatur: 70 bis 90 °C; Kopfdruck: 150 bis 190 mbar;
- Sumpftemperatur: 100 bis 120 °C; Sumpfdruck: 250 bis 350 mbar.

Rektifikationseinheit III:
- Kopftemperatur: 70 bis 90 °C; Kopfdruck: 90 bis 120 mbar;
- Sumpftemperatur: 100 bis 120 °C; Sumpfdruck: 150 bis 180 mbar.

Der in der Rektifikationseinheit III verbleibende, im wesentlichen noch Zielester enthaltende, sowie aus Butoxyester, michaeloligomere Acrylsäurebutylester, Polymerisationsinhibitor sowie radikalisch oligo- und/oder polymerisierten n-Butyl-acrylaten zusammengesetzte hochsiedende Rest (Sumpfprodukt), kann zum Zweck der weiteren Ausbeutesteigerung einem Rückspaltungsverfahren, mit Vorteil einem wie in der DE-A 197 01 737, oder der DE-A 195 36 191, oder der DE-A 195 36 184, oder der DE-A 195 47 485, oder der DE- 30 A 195 47 459, oder der CN-A 1 063 678 beschriebenen Rückspaltungsverfahren, der Butoxyester und michaeloligomeren Acrylsäurebutylester unterworfen und erfindungsgemäß verarbeitet werden. Der bei der Rückspaltung verbleibende hochsiedende Rest (hochsiedend in Bezug auf das Verfahrensprodukt n-Butylacrylat) wird abschließend entsorgt, z. B. verbrannt.

Die typischen Formeln der Butoxyester und michaeloligomeren Acrylsäurebutylester sind unten beim Beispiel 1 aufgeführt.

Charakteristisch für die Butoxyester und michaeloligomeren Acrylsäurebutylester ist, dass ihre Siedepunkte oberhalb der Siedepunkte von Acrylsäure, n-Butanol und gebildetem n-Butyl-acrylat liegen. Wesentlich ist, dass die Butoxyester- Bildung und michaeloligomere Acrylsäurebutylester-Bildung reversibel ist. D. h., durch die Einwirkung erhöhter Temperatur im Beisein saurer Spaltkatalysatoren können die Butoxyester und michaeloligomeren Acrylsäurebutylester in ihre Ausgangsverbindungen zurückgespalten werden.

Im Folgenden ist ferner von Bedeutung, dass Acrylsäure auch mit sich selbst reversibel Michael-addieren kann. Die dabei resultierenden Oligomere der Acrylsäure sollen im Folgenden zur Unterscheidung von durch radikalische Polymerisation gebildeten Oligomeren als Michael-Oligomere bezeichnet werden. Michael-oligomere Acrylsäure fällt z.B. bei der destillativen Behandlung von roher Acrylsäure im Sumpf an (vgl. z.B. DE-A 22 35 326). Michael-oligomere Acrylsäure lässt sich durch die folgende allgemeine Formel charakterisieren:

CH₂=CH-CO₂-[CH₂-CH₂-CO₂]_{z}-H

mit z = eine ganze Zahl ≥ 1, häufig 1 bis 5.

Geeignete Butoxyester und michaeloligomere Acrylsäurebutylester Rückspaltkatalysatoren sind, wie weiter oben und besonders auch in DE 198 51 983 A1 (BASF AG) beschrieben, z.B. Mineralsäuren wie Schwefelsäure oder Phosphorsäure sowie organische Säuren wie Methansulfonsäure, p-Toluolsulfonsäure, Dodecylbenzolsulfonsäure (CAS-Nummer: 27176-87-0) oder Maranil^{®} DBS/LC (CAS-Nummer: 85536-14-7). Die Rückspaltung der im Sumpfprodukt der Rektifikationseinheit III enthaltenen Butoxyester und michaeloligomeren Acrylsäurebutylester ist daher in einfacher Weise dadurch möglich, dass man dem Sumpfprodukt wenigstens einen sauren Spaltkatalysator zusetzt und anschließend das Gemisch den weiter oben genannten Bedingungen unterwirft, so dass die Spaltprodukte unmittelbar abdampfen und unmittelbar, gegebenenfalls nach Kondensation und mit Polymerisationsinhibitoren stabilisiert, in erfindungsgemäßer Weise in die Reaktionszone rückgeführt bzw. ausgeschleust werden können.

Im Übrigen kann die Rückspaltung gemäß der Lehre der DE-A 195 47 459 bzw. der DE-A 195 47 485 im zusätzlichen Beisein von monomerer Acrylsäure und/oder michaeloligomerer Acrylsäure in den bereits oben genannten Mengen durchgeführt werden.

Normalerweise erfolgt der Zusatz der monomeren und/oder oligomeren Acrylsäure zu der Spaltung zu unterwerfenden Sumpfprodukt in an sich bekannter, mittels Polymerisationsinhibitoren stabilisierter, Form. Als oligomere Acrylsäure kann dabei in besonders einfacher Weise, der bei der destillativen Reinigung von Roh-Acrylsäure anfallende Sumpf eingesetzt werden, der hauptsächlich Verbindungen der allgemeinen Formel

CH₂=CH-CO₂-[CH₂-CH₂-CO₂]_{z}-H,

mit z = eine ganze Zahl ≥ 1, häufig 1 bis 5,
enthält (siehe z.B. DE-A 22 35 326). Ein Beisein von monomerer und/oder michaeloligomerer Acrylsäure bei der Rückspaltung bewirkt vor allem eine erhöhte Spaltgeschwindigkeit und eine reduzierte Ether- und Olefin-Nebenproduktbildung.

Zusätzlich kann, gemäß der Lehre der DE-A 197 01 737, die Rückspaltung des Sumpfproduktes im Beisein von Wasser vorgenommen werden. Bezogen auf die Menge der zu spaltenden Butoxyester und michaeloligomeren Acrylsäurebutylester beträgt vorgenannte Wassermenge in der Regel 0,1 bis 20 Gew.-%, häufig 1 bis 10 Gew.-%.

Das zu spaltende Sumpfprodukt und die zur Spaltung zu verwendenden Säuren (schwefelhaltige mineralische Säure, phosphorhaltige mineralische Säure, organische Sulfonsäure) sowie gegebenenfalls monomere und/oder michaeloligomere Acrylsäure und gegebenenfalls Wasser können dem zu spaltenden Sumpfprodukt bereits vor dessen Überführung in den Spaltreaktor zugegeben werden. Sie können dem Spaltreaktor aber auch getrennt zugeführt werden. Bei einem Teil oder der Gesamtmenge der erforderlichen sauren Spaltkatalysatoren kann es sich auch um die sauren Veresterungskatalysatoren handeln. Gemäß einer vorteilhaften Ausbildung der Erfindung wird die Rückspaltung im Beisein von molekularem Sauerstoff durchgeführt.

Besonders günstig ist, wenn durch das zu spaltende Gemisch im Rahmen des erfindungsgemäßen Verfahrens als Schleppmittel für die Spaltprodukte ein Strippgas, dass vorzugsweise molekularen Sauerstoff enthält, geführt wird. Zweckmäßigerweise werden als Strippgas Luft oder Gemische von Luft mit Inertgas (z.B. Stickstoff) verwendet.

Wird während der Rückspaltung durch das Spaltgemisch ein Strippgas geführt, beträgt in einer kontinuierlichen Fahrweise dessen Menge im Normalfall 1 bis 100 Liter pro Stunde pro Liter Sumpfprodukt. Im Regelfall erfordert die Rückspaltung Reaktionsdauern im Bereich von 1 bis 15 h. Der Umsatz der Rückspaltung beträgt im Normalfall > 90 Gew.-%. Zur Durchführung der Rückspaltung kann z.B. ein einfacher beheizbarer Rührreaktor mit Doppelwandheizung oder Heizschlange oder auch ein Zwangsumlaufverdampfer, beispielsweise ein Fallfilmverdampfer oder Flashverdampfer, gekoppelt mit einem Verweilzeitbehälter, verwendet werden. Zur besseren Trennung der Spaltprodukte vom Sumpfprodukt ist gegebenenfalls eine auf die Spaltapparatur aufgesetzte Rektifikationsvorrichtung, z.B. eine Füllkörper-, Packungs- oder Bodenkolonne, zweckmäßig. Diese Rektifikationsvorrichtung wird in der Regel mit Polymerisationsinhibitoren (z.B. Phenothiazin, Hydrochinonmonomethylether, Hydrochinon etc.) stabilisiert betrieben. Bevorzugt ist auch das rückzuspaltende Sumpfprodukt von der Veresterung herrührend mittels Polymerisationsinhibitoren polymerisationsstabilisiert.

Die Reaktionsführung der Rückspaltung läuft z.B. in der Weise, dass das zu spaltende Sumpfprodukt kontinuierlich aus der Rektifikationseinheit III ausgeschleust und mit den erforderlichen Spaltkatalysatoren, gegebenenfalls monomerer und/oder michaeloligomerer Acrylsäure sowie gegebenenfalls Wasser dem Spaltreaktor zugeführt wird. Die Reaktionsführung kann aber auch diskontinuierlich, d. h. batchweise, durchgeführt werden. Auch eine halbkontinuierliche Reaktionsführung ist möglich, bei der das zu spaltende Sumpfprodukt sowie die diesem gegebenenfalls zuzugebenden Zusätze kontinuierlich dem Spaltreaktor, der den sauren Spaltkatalysator enthält, zugeführt und das bei der Spaltung anfallende Sumpfprodukt erst nach Beendigung der Spaltung aus dem Spaltreaktor batchweise entfernt wird.

Die bei der Rückspaltung gasförmig entweichenden Rückspaltprodukte (besonders n-Butanol, n-Butylacrylat, Di-n-butyl-ether und Acrylsäure) und Wasserdampf werden zunächst kondensiert und dann einer Rektifikationseinheit V zugeführt (Strom 4), in der Wasser, n-Butanol und Di-n-butyl-ether abdestilliert werden, und aus der das Sumpfprodukt in die Reaktionszone rückgeführt wird (Strom 5).

Selbstredend kann das Rückspaltungsverfahren auch mehrstufig, z.B. zweistufig, (z. B. in einer Kaskade, z. B. wie in der CN 1063678 A beschrieben) durchgeführt werden.

Die Verweilzeit in den einzelnen Stufen kann dabei gleich oder verschieden sein. Vorzugsweise nimmt sie von der ersten zur letzten Stufe hinzu. Bei einer zweistufigen Durchführung beträgt die Verweilzeit des Spaltgemisches in der ersten Stufe zweckmäßigerweise 1 bis 15 h und in der zweiten Stufe 10 bis 40 h. Bei einer einstufigen, bevorzugt kontinuierlichen, Durchführung beträgt die Verweilzeit des Spaltgemisches bevorzugt 4 bis 14 h, besonders bevorzugt 6 bis 12 h.

Ferner nimmt die Rückspalttemperatur bei einer mehrstufigen Ausführweise vorzugsweise zur letzten Stufe hinzu.

Der Vorteil der vorstehend beschriebenen stufenförmigen Spaltung besteht darin, dass das bei der Veresterung anfallende Sumpfprodukt in der Regel noch signifikante Mengen des Zielesters enthält, die bei den hohen Spalttemperaturen besonders (radikalisch) polymerisationsanfällig und auch gegen die sauren Spaltkatalysatoren nicht inert sind. Bei vergleichsweise milden Spaltbedingungen in der ersten Stufe können diese Zielesteranteile im Wesentlichen unverändert mit den entstehenden Spaltprodukten schonend abgetrennt werden, bevor die Rückspaltung in den nachfolgenden Stufen unter Verschärfung der Spaltungsbedingungen vervollständigt werden kann. Bei einer kontinuierlichen Durchführung einer mehrstufigen Spaltung kann der Druck in aufeinanderfolgenden Stufen gleich oder verschieden sein. Im Fall eines konstanten Druckes kann der Transport von der einen zur anderen Stufe in einfacher Weise durch standgeregelten Überlauf erfolgen. Bei unterschiedlichen Drucken empfiehlt sich ein gepumpter Gemischtransport.

Der erfindungsgemäß hergestellte Acrylsäure-n-butylester findet besonders vorteilhaft Verwendung in der Herstellung von Kontaktlinsen oder als Vernetzer oder Haftverbesserer für Dispersionen, die vorzugsweise als Klebstoffe, Anstrichmittel, Farben, z.B. auch Druckfarben, oder Textil-, Leder- oder Papierhilfsmittel sowie in härtbaren Beschichtungen eingesetzt werden.

Di-n-butyl-ether (gekennzeichnet nach GHS; H 412 - giftig für Wasserorganismen), stellt eine zunehmend kritische Verunreinigung des n-Butylacrylats dar. n-BA dient hauptsächlich als Rohstoff für ist die Herstellung der Dispersionen, die wiederum eine Hauptkomponente bei der Herstellung von Materialien für architektonische Beschichtungen, besonders Innen- und Außenanstriche, darstellen. Diese Produkte fallen seit Jahren stärker in den Fokus der Regulierungsbehörden.

Z.B. erlaubt der Beschluss der EU Kommission 2014/312/EU zur Vergabe des EU-Umweltzeichens für Innen- und Außenfarben und -lacke nicht das Vorhandensein in einer Konzentration von über 100 ppm von halbflüchtigen und flüchtigen organischen Verbindungen (Volatile Organic Compound(s) = VOC), wie DBE, in der endgültigen Farbformulierung, die, neben vielen anderen H-Sätzen, als H412 eingestuft sind. Das bedeutet, dass Polymerdispersionen mit mehr als 100 ppm DBE für die Vergabe vieler Umweltzeichen ausgeschlossen sind.

Die Bedeutung von Umweltzeichen wie dem "EU-Umweltzeichen", dem "Nordischen Schwan" oder dem "Blauen Engel" nimmt in der europäischen Architekturbeschichtungsindustrie deutlich zu. Diese Zeichen definieren "Umweltfreundlichkeit" der Produkte, geben eine Auskunft über qualitative Unterschiede der auf dem Markt erhältlichen Produkte und beeinflussen damit wesentlich die Kaufentscheidungen und Verhalten der Endkunden.

In diesem Zusammenhang ist auch zu erwähnen, dass die Erwartung gegenüber der n-BA Hersteller den gesamten VOC-Gehalt in ihrem Produkt zu reduzieren, zunimmt. Dies gilt nicht nur für das Segment der Architekturbeschichtungen, sondern auch für viele andere, bei denen n-BA das Hauptmonomer (z.B. Konstruktion, Kunststoffzusätze) verwendet wird.

Alle Druckangaben beziehen sich auf dem Absolutdruck.

Alle ppm-Angaben beziehen sich auf das Gewicht (Gew.-ppm).

### Beispiele

### Beispiel 1 (nicht gemäß Anspruch 1)

In einer großtechnischen Anlage erfolgte die Veresterung von Acrylsäure mit n-Butanol in drei, in der Reihe aufgestellten Reaktionsbehältern (= drei Reaktionsbereiche). Die Einsatzstoffe wurden in den ersten Reaktionsbehälter kontinuierlich vorgegeben. Die Energiezufuhr und die Durchmischung des Reaktionsgemisches erfolgten durch die für jeden Reaktionsbehälter separaten Naturumlaufverdampfer. Die Abtrennung des Veresterungswassers fand in den auf den Reaktionsbehältern aufgesetzten Kolonnen (Veresterungswasser ist in dem System ein Leichtsieder) statt. Die Synthese wurde unter einem Druck im Bereich von 500 bis 600 mbar (absolut) betrieben. In den Destillattrennbehältern bildete das Leichtsieder-Wasser-Gemisch zwei Phasen. Die organische Phase mit den Leichtsiedern wurde in die Kolonnen als Rücklauf gefahren und ein kleiner Teil wurde ausgeschleust, um die Anreicherung der Leichtsieder im System zu verhindern.

Der erste Reaktionsbehälter wurde mit einem AS-Zulauf (AS = Acrylsäure) von 6 t/h betrieben. Die n-Butanolmenge wurde in den ersten Reaktionsbehälter so dosiert, dass ein n-Butanol zu Acrylsäure Massenverhältnis von 1,13 eingestellt wurde. Die Temperatur in den Reaktionsbehältern wurde im Bereich von 114 °C in dem ersten Reaktionsbehälter bis 120 °C in dem dritten Reaktionsbehälter eingestellt.

Die Konzentration der Schwefelsäure in den Reaktionsbehältern wurde von 1,6 bis 1,7 Gew.-% (bezogen auf den Auslauf (Abzug) aus dem dritten Reaktionsbehälter) langsam auf 1,1 Gew.-% erniedrigt. Die standardmäßig durchgeführten Analysen zeigten einen Anstieg an Acrylsäure im Überlauf des dritten Reaktionsbehälters von 0,9 bis auf 1,2 bis 1,3 Gew.-% (bezogen auf den Auslauf (Abzug) aus dem dritten Reaktionsbehälter). Gleichzeitig sank die Konzentration an Butoxyester und michaeloligomerer Acrylsäurebutylester von 4,5 bis auf 3,5 Gew.-% (bezogen auf den Zulauf zur Rektifikationseinheit II), während die Konzentration an n-BA (n-BA = n-Butylacrylat) unverändert blieb. Eine erneute Erhöhung der Schwefelsäure-Konzentration in der Synthese rief eine Absenkung des Gehalts an AS im Überlauf des dritten Reaktionsbehälters und ein Ansteigen der BOE Konzentration (BOE = Butoxyester + michaeloligomere Acrylsäurebutylester) hervor. Siehe die folgende Tabelle:

| Zugesetzte H₂SO₄-Menge [Gew.-%] | Zugesetzte H₂SO₄-Menge [mmol Säure pro kg aus der Reaktionszone abgezogenem Reaktionsgemisch] | Acrylsäure Konzentration [Gew.-%] | Butoxyester und michaeloligomere Acrylsäurebutylester [Gew.-%] | n-BA [Gew.-%] |
|---|---|---|---|---|
| 1,7 - 1,6 | 173 - 164 | 0,9 - 1,0 | 4,1 - 4,5 | 87 - 88 |
| 1,4 - 1,5 | 143 - 153 | 1,05 - 1,15 | 4,1 - 4,2 | 87 - 88 |
| 1,1 - 1,3 | 112 - 133 | 1,1 - 1,2 | 3,8 - 4,0 | 87 - 88 |
| 1,1 | 112 | 1,2 - 1,3 | 3,5 - 3,8 | 87 - 88 |

Die Gew.-% Angaben in der Tabelle in Spalte 1 und 3 sind jeweils bezogen auf die Menge an aus der Reaktionszone abgezogenem Reaktionsgemisch.

Die Gew.-% Angaben in der Tabelle in Spalte 4 und 5 sind jeweils bezogen auf den Zulauf zur Rektifikationseinheit II.

Butoxyester bzw. michaeloligomere Acrylsäurebutylester sind Verbindungen der folgenden Formeln (wie z.B. auch in EP 780359 B1, Absatz [0002], und in DE 198 51 983 A1, Seite 7, beschrieben):

RO-[CH₂-CH(R')-CO₂]ₓ-R,

H₂C=C(R')-CO₂-[CH₂-CH(R')-CO₂]_{y}-R,

mit R = n-Butyl, R' = H, x, y = eine ganze Zahl ≥ 1, besonders im Bereich von 1 bis 5.

### Beispiel 2 (nicht gemäß Anspruch 1)

Die organische Phase aus der Vorreinigung III wurde einer zweistufigen Destillation unterzogen. Im ersten Schritt wurden in der Rektifikationseinheit II die Leichtsieder über den Kopf der Kolonne (Wasser, n-Butanol, Butylacetat und Di-n-butyl-ether), in der Rektifikationseinheit III Reinprodukt ebenfalls über Kopf der Kolonne abgezogen. Das Sumpfprodukt der Rektifikationseinheit III lief mengengeregelt in einen Pufferbehälter (3000 kg/h). Von dort wurde es Rührspaltkesseln zugeführt. In den Rührspaltkesseln wurden bei erhöhter Temperatur (180 °C) unter Zugabe von Maranil^{®} DBS/LC (12 % bezogen auf das Reaktionsgemisch der Rückspaltung) (CAS-Nummer: 85536-14-7) und Acrylsäure (15 Gew.-% bezogen auf den Sumpfablauf der Rektifikationseinheit III bzw. bezogen auf Zulauf der Spaltung), Butoxyester, michaeloligomere Acrylsäurebutylester und evtl. andere langkettige Schwersieder in n-Butylacrylat und n-Butanol gespalten. Das freigesetzte n-Butanol wurde direkt, in situ, mit Acrylsäure zu n-BA verestert.

Die Zulaufmenge in die Spaltkessel wurde bei 3000-3100 kg/h konstant gehalten.

Die Konzentration und damit auch die Fracht an Butoxyester und michaeloligomerer Acrylsäurebutylester in die Rückspaltung (VI) wurde durch Änderung der H2SO4-Konzentration in der Veresterung, gemäß Beispiel 1, von 56 Gew.-% bis auf 49 Gew.-% (bezogen auf den Zulauf in den Rührspaltkessel) erniedrigt.

Das Wertprodukt wurde aus den Spaltkesseln über eine Füllkörperkolonne abdestilliert. Das kondensierte Produkt wurde im Destillatbehälter gesammelt. Von dort wurde ein Teilstrom zu den Kolonnen der Rückspaltungen als Rücklauf zurückgeführt.

Das Destillat (Strom 4) aus den Spaltkesseln (3100-3200 kg/h) wurde zurück zu der Rektifikationseinheit I gefahren.

Durch Erniedrigung der BOE-Konzentration in den Reaktionsbehältern und somit auch des BOE-Mengenstroms zur Rückspaltung VI wurde die Di-n-butyl-ether-Konzentration im Destillat der Rückspaltung VI und somit auch dessen Fracht in die Veresterung erniedrigt - siehe die folgende Tabelle:

| Tag | BOE Konzentration im Feed zur Rückspaltung [Gew.-%] | DBE im Destillat (Strom 4) [ppm] |
|---|---|---|
| 1 | | 7661 |
| 2 | | - |
| 3 | | - |
| 4 | | - |
| 5 | | 6372 |
| 6 | | 6406 |
| 7 | | 7349 |
| 8 | 56 | 6779 |
| 9 | | 6520 |
| 10 | | |
| 11 | | |
| 12 | | 6508 |
| 13 | | |
| 14 | | 5730 |
| 15 | | 5470 |
| 16 | 51,7 | 5150 |
| 17 | | |
| 18 | | |
| 19 | | 4837 |
| 20 | 48,9 | 4713 |
| 21 | | 4940 |
| 22 | | 5234 |
| 23 | 50,7 | 5148 |
| 24 | | |
| 25 | | |
| 26 | | 5422 |
| 27 | 52,4 | 6061 |
| 28 | | 6370 |

### Beispiel 3 (nicht gemäß Anspruch 1)

Das Destillat (Strom 4) aus der Rückspaltung VI aus dem Beispiel 2 (3000-3100 kg/h) wurde in die Reaktionsbehälter zurückgeführt. Der Aufbau und Funktionsweise der Reaktionsbehälter erfolgte analog zur Beschreibung in Beispiel 1. In den ersten Reaktionsbehälter wurde AS in der Menge von 11 t/h und n-Butanol hineingefahren, so dass ein n-Butanol zu AS Gewichtsverhältnis von 1,13 eingestellt wurde. In der Synthese wurde die Schwefelsäure-Konzentration allmählich erniedrigt von 1,6 auf unter 1,3 Gew.-% (bezogen auf den Auslauf (Abzug) des dritten Reaktionsbehälters). Der Rohester wurde einer destillativen Aufarbeitung gemäß Beispiel 2 unterzogen. Die Leichtsieder aus der Rektifikationseinheit II wurden zurück in die Synthese gefahren und der Sumpf wurde zu der Rektifikationseinheit III gefahren.

Rektifikationseinheit II, bestehend aus einer Kolonne mit 60 Dual-Flow Böden, einem Verdampfer, Kondensator und Rücklaufbehälter mit Phasenscheider, wurde kontinuierlich (Zulauf auf den 37. Boden) mit der organischen Phase aus der Vorreinigung III versorgt. Die Rektifikationsbedingungen, Destillatabzug und Sumpfabzug wurden so angepasst, dass bei einem Druck von 120 mbar über Kopf der Kolonne Wasser, Butylacetat, ein Teil des Di-n-butyl-ethers mit möglichst wenig n-BA abgezogen wurden - Kolonnenkopftemperatur 60-70 °C, Sumpftemperatur 100-110 °C.

Das kondensierte Kopfprodukt wurde im Phasenscheider in eine wässrige und eine organische Phase getrennt und ein Teil der organischen Phase wurde als Kopfprodukt auf die Kolonne zurückgefahren (Rücklaufverhältnis 7 bis 9). Sumpfprodukt der Rektifikationseinheit II wurde auf den 10. Boden der Rektifikationseinheit III kontinuierlich gefahren.

Rektifikationseinheit III umfasste eine Kolonne mit 20 Dual-Flow Böden, einen Verdampfer, Kondensator und Rücklaufbehälter. Die Rektifikationsbedingungen, Destillatabzug und Sumpfabzug wurden so angepasst, dass bei einem Druck von 120 mbar über Kopf der Kolonne Reinester abgezogen wurde - Kolonnenkopftemperatur 80 bis 85 °C; Sumpftemperatur 105 bis 110 °C.

Ein Teil der kondensierten Brüden wurde als Rücklauf auf die Kolonne zurückgefahren (Rücklaufverhältnis 0,3 bis 0,7).

Als Destillat wurde das n-BA-Reinprodukt abgezogen, der Sumpf wurde in der Rückspaltung VI gemäß Vergleichsbeispiel 2 aufgearbeitet. Durch die Variation der H2SO4-Konzentration in der Synthese (Beispiel 1) konnte Einfluss auf die BOE-Bildung und DBE-Bildung genommen werden und somit konnte auch im n-BA-Reinprodukt die DBE-Konzentration wesentlich erniedrigt werden. Siehe die Grafik 1.

### Beispiel 4

Rektifikationseinheit IV (thermodynamische Simulation: DBE-Abtrennung aus dem Destillat der Rektifikationseinheit II)

Die Fahrweise wird anhand der Daten aus einer thermodynamischen Simulation einer Gesamtanlage (Figur 1) zur Herstellung von n-Butylacrylat dargestellt.

Die thermodynamische Simulation des Prozesses wurde mit der Software Aspen Plus^{®} (kurz: Aspen) durchgeführt. Aspen ist eine umfangreiche Simulationssoftware, die zur Modellierung, Simulation und Optimierung chemischer Verfahren und Anlagen in der Industrie eingesetzt wird. Aspen verfügt über umfangreiche Modell-Datenbanken zur Modellierung der Basisoperationen sowie über Stoffdatenbanken für die Stoffeigenschaften vieler verschiedener Substanzen. Die Eigenschaften von Mischungen werden von Aspen mit Hilfe von unterschiedlichen thermodynamischen Modellen aus den Stoffdaten der reinen Substanzen berechnet.

Die Nebenkomponenten n-Butylacetat und Di-n-butyl-ether können aus dem Wertprodukt entfernt werden, indem der im Gesamtverfahren zurückgeführte Destillatstrom (1) aus der Rektifikationseinheit II destillativ aufgearbeitet wird.

Die thermodynamische Simulation der Rektifikationseinheit IV zur Abtrennung der Nebenkomponenten n-Butylacetat und Di-n-butyl-ether aus dem Destillatstrom der Rektifikationseinheit II führte zu folgenden Ergebnissen:
Ein Roh-Butylacrylat von 1000 kg/h mit einer Temperatur von 40 °C wird auf der 10. theoretischen Trennstufe in eine Rektifikationskolonne mit 20 theoretischen Trennstufen aufgegeben.

Die Zusammensetzung des zulaufenden Destillatstromes (1) aus der Rektifikationseinheit II beträgt:

| | |
|---|---|
| Wasser | 5,7 Gew.-% |
| n-Butanol | 32,2 Gew.-% |
| n-Butylacetat | 0,9 Gew.-% |
| n-Butylacrylat | 60,4 Gew.-% |
| Di-n-butyl-ether | 0,8 Gew.-% |

Der Betrieb der Kolonne erfolgt bei einem Kopfdruck von 160 mbar und einem Sumpfdruck von 274 mbar. Der spezifische Druckverlust der Kolonne beträgt 5,7 mbar/theoretischer Trennstufe. Die Kopftemperatur beträgt 51 °C. Am Kopf der Kolonne wird das Destillat kondensiert. Der Abzugsstrom an Destillat beträgt 146 kg/h. Die Kolonne wird bei einem Rücklaufverhältnis von 6,4 betrieben.

Die Zusammensetzung des Destillates (3) beträgt:

| | |
|---|---|
| Wasser | 39,0 Gew.-% |
| n-Butanol | 46,3 Gew.-% |
| n-Butylacetat | 5,6 Gew.-% |
| n-Butylacrylat | 5,0 Gew.-% |
| Di-n-butyl-ether | 4,1 Gew.-% |

Der Verdampfer wird dabei mit 429 kW betrieben. Das aus dem Verdampfer auslaufende Sumpfprodukt von 854 kg/h hat dabei eine Temperatur von 91 °C.

Die Zusammensetzung des Sumpfablaufs (2) beträgt:

| | |
|---|---|
| Wasser | 0,0 Gew.-% |
| n-Butanol | 29,9 Gew.-% |
| n-Butylacetat | 0,0 Gew.-% |
| n-Butylacrylat | 69,9 Gew.-% |
| Di-n-butyl-ether | 0,2 Gew.-% |

In einem Verfahren gemäß Figur 2 beträgt die Konzentration an Nebenkomponenten im Wertprodukt:

| | |
|---|---|
| n-Butylacetat | 600 Gew.-ppm |
| Di-n-butyl-ether | 1200 Gew.-ppm |

Gegenüber dem bestehenden Destillationsverfahren kann durch das erfindungsgemäße Verfahren unter Einsatz der Rektifikationskolonne IV die Konzentration an Nebenkomponenten im Wertprodukt bei gleicher Tageskapazität auf folgende Werte reduziert werden.

| | |
|---|---|
| n-Butylacetat | 15 Gew.-ppm |
| Di-n-butyl-ether | 260 Gew.-ppm |

Beispiel 5: Rektifikationseinheit IV und Rektifikationseinheit V (thermodynamische Simulation: DBE-Abtrennung aus dem Destillat der Rektifikationseinheit II und zusätzliche DBE-Abtrennung aus dem Destillat der Rückspaltung)

Die Fahrweise wird anhand der Daten aus einer thermodynamischen Simulation einer Gesamtanlage (Figur 1) zur Herstellung von n-Butylacrylat dargestellt.

Die thermodynamische Simulation des Prozesses wurde mit der Software Aspen Plus^{®} (kurz: Aspen) durchgeführt. Aspen ist eine umfangreiche Simulationssoftware, die zur Modellierung, Simulation und Optimierung chemischer Verfahren und Anlagen in der Industrie eingesetzt wird. Aspen verfügt über umfangreiche Modell-Datenbanken zur Modellierung der Basisoperationen sowie über Stoffdatenbanken für die Stoffeigenschaften vieler verschiedener Substanzen. Die Eigenschaften von Mischungen werden von Aspen mit Hilfe von unterschiedlichen thermodynamischen Modellen aus den Stoffdaten der reinen Substanzen berechnet.

Um gegenüber dem Beispiel 4 eine noch bessere Abtrennung der Nebenkomponente Di-n-butyl-ether aus dem Wertprodukt zu erhalten, kann zusätzlich der aus der Rückspaltung zurückgeführte Destillatstrom (4) destillativ aufgearbeitet werden.

Die thermodynamische Simulation der Rektikationseinheit zur Abtrennung der Nebenkomponente Di-n-butyl-ether aus dem Destillatstrom (4) der Rückspaltung führte zu folgenden Ergebnissen:
Ein Destillatstrom (4) aus der Rückspaltung von 684 kg/h mit einer Temperatur von 40 °C wird auf der 8. theoretischen Trennstufe in eine Rektifikationskolonne mit 20 theoretischen Trennstufen aufgegeben.

Die Zusammensetzung des Destillatstromes (4) aus der Rückspaltung beträgt:

| | |
|---|---|
| Wasser | 3,0 Gew.-% |
| n-Butanol | 5,2 Gew.-% |
| Acrylsäure | 4,4 Gew.-% |
| n-Butylacrylat | 77,0 Gew.-% |
| Di-n-butyl-ether | 1,2 Gew. -% |
| Butoxyester + michaeloligomere Acrylsäurebutylester | 9,2 Gew.-% |

Der Betrieb der Kolonne erfolgt bei einem Kopfdruck von 160 mbar und einem Sumpfdruck von 230 mbar. Der spezifische Druckverlust der Kolonne beträgt 3,5 mbar/theoretischer Trennstufe. Die Kopftemperatur beträgt 55 °C. Am Kopf der Kolonne wird das Destillat kondensiert. Der Abzugsstrom an Destillat (6) beträgt 100 kg/h. Die Kolonne wird bei einem Rücklaufverhältnis von 9,4 betrieben.

Die Zusammensetzung des Destillates (6) beträgt:

| | |
|---|---|
| Wasser | 20,6 Gew.-% |
| n-Butanol | 35,5 Gew.-% |
| Acrylsäure | 0,0 Gew.-% |
| n-Butylacrylat | 36,4 Gew.-% |
| Di-n-butyl-ether | 7,5 Gew.-% |
| Butoxyester + michaeloligomere Acrylsäurebutylester | 0,0 Gew.-% |

Der Verdampfer wird dabei mit 293 kW betrieben. Das aus dem Verdampfer auslaufende Sumpfprodukt von 584 kg/h hat dabei eine Temperatur von 102°C.

Die Zusammensetzung des Sumpfablaufs (5) beträgt:

| | |
|---|---|
| Wasser | 0,0 Gew.-% |
| n-Butanol | 0,0 Gew.-% |
| Acrylsäure | 5,2 Gew.-% |
| n-Butylacrylat | 83,9 Gew.-% |
| Di-n-butyl-ether | 0,1 Gew.-% |
| Butoxyester + michaeloligomere Acrylsäurebutylester | 10,8 Gew.-% |

In einem Verfahren gemäß Figur 2 beträgt die Konzentration an Di-n-butyl-ether im Wertprodukt ca. 1200 Gew.-ppm. Die Konzentration an Di-n-butyl-ether im Wertprodukt beträgt nach dem erfindungsgemäßen Beispiel 4 ca. 260 Gew.-ppm.

Gegenüber dem Destillationsverfahren gemäß Figur 2 wird durch das erfindungsgemäße Verfahren unter Einsatz der Rektifikationseinheiten IV + V die Konzentration an Di-n-butyl-ether im Wertprodukt bei gleicher Tageskapazität auf ca. 150 Gew.-ppm reduziert.

## Patentansprüche

1. Verfahren zur kontinuierlichen Herstellung von Acrylsäure-n-butylester durch Umsetzung von Acrylsäure mit n-Butanol in an Lösungsmittel freier Phase bei erhöhter Temperatur und unter Zusatz von Säure als Veresterungskatalysator, bei dem man die Acrylsäure, das n-Butanol und den Veresterungskatalysator einer Reaktionszone zuführt, während einer Verweilzeit in der Reaktionszone das gebildete Wasser als Bestandteil eines n-Butanol umfassenden Gemisches in einer der Reaktionszone aufgesetzten ersten Rektifikationseinheit I vom Reaktionsgemisch abtrennt, das dabei anfallende Destillat in eine n-Butanol enthaltende organische und in eine Wasser enthaltende wässrige Phase auftrennt, die organische Phase in die Rektifikationseinheit I zurückführt, die wässrige Phase gegebenenfalls, ganz oder teilweise, einer Strippeinheit IX zuführt, das den Acrylsäure-n-butylester enthaltende Reaktionsgemisch, das aus der Reaktionszone abgezogen wird, einer Vorreinigung zuführt, wobei
1. in einer ersten Vorreinigungsstufe (Vorreinigung I) der überwiegende Teil des Veresterungskatalysators mittels Wasserwäsche extraktiv abgetrennt und
2. in einer zweiten Vorreinigungsstufe (Vorreinigung II) die sauren Komponenten mit einer wässrigen Alkalilösung durch Reaktivextraktion neutralisiert und extrahiert werden und
3. optional, in einer dritten Vorreinigungsstufe (Vorreinigung III), aus dem nach der zweiten Vorreinigungsstufe verbleibenden organischen Reaktionsrestgemisch restliche Salze sowie wässrige Fremdphasenanteile mit Wasser extraktiv entfernt werden,
das dabei verbleibende organische Reaktionsrestgemisch I in eine weitere Rektifikationseinheiten umfassende Trennzone leitet und in dieser den gebildeten n-Butylester der Acrylsäure abtrennt, indem man
- das verbleibende Reaktionsrestgemisch I einer Rektifikationseinheit II zuführt und in dieser das verbliebene Reaktionsrestgemisch I in ein Acrylsäure-n-butylester und leichter als der Acrylsäure-n-butylester siedende Bestandteile enthaltendes Leichtsiederprodukt und in ein den Acrylsäure-n-butylester und schwerer als der Acrylsäure-n-butylester siedende Bestandteile umfassendes Reaktionsrestgemisch II rektifikativ auftrennt,
- das Reaktionsrestgemisch II einer Rektifikationseinheit III zuführt und in dieser den Acrylsäure-n-butylester von den schwerer als der Acrylsäure-n-butylester siedenden Bestandteilen abtrennt,
**dadurch gekennzeichnet, dass**
- die Menge an zugesetzter Säure als Katalysator in die Reaktionszone im Bereich von 51 bis 163 mmol Säure pro kg des aus der Reaktionszone abgezogenen Reaktionsgemischs beträgt,
- man n-Butanol und Acrylsäure in einem Massenverhältnis im Bereich von n-Butanol : Acrylsäure = 1,0 bis 1,3 einsetzt,
- man wenigstens den organischen Anteil des nicht als Rücklauf in die Rektifikationseinheit II verwendeten Leichtsiederproduktes aus der Rektifikationseinheit II zumindest teilweise einer Rektifikationseinheit IV zuführt (Strom 1), in der Wasser und n-Butanol und n-Butylacetat und Di-n-butyl-ether abdestilliert werden, das Destillat (Strom 3) zumindest teilweise einer n-Butanol-Extraktionseinheit VII zuführt und das Sumpfprodukt (Strom 2) entweder direkt oder über eine Acrylsäure-Extraktionseinheit VIII in die Reaktionszone rückführt.

2. Verfahren nach Anspruch 1 **dadurch gekennzeichnet, dass**
- das in der Rektifikationseinheit III anfallende Sumpfprodukt einer Rückspaltung VI im Beisein einer schwefelhaltigen mineralischen Säure, phosphorhaltigen mineralische Säure oder organischen Sulfonsäure bei erhöhter Temperatur unterworfen wird und die dabei gasförmig entweichenden Rückspaltprodukte zunächst kondensiert und dann einer Rektifikationseinheit V zuführt (Strom 4), in der Wasser, n-Butanol und Di-n-butyl-ether abdestilliert werden, und aus der das Sumpfprodukt in die Reaktionszone rückgeführt wird (Strom 5).

3. Verfahren nach dem vorhergehenden Anspruch, **dadurch gekennzeichnet, dass** es sich bei der organischen Sulfonsäure um eine aromatische Sulfonsäure handelt.

4. Verfahren nach dem vorhergehenden Anspruch, **dadurch gekennzeichnet, dass** es sich bei der aromatischen Sulfonsäure um Dodecylbenzolsulfonsäure oder Maranil^{®} DBS/LC (CAS-Nummer: 85536-14-7) handelt.

5. Verfahren nach einem der vorhergehenden Ansprüche 2 bis 4, **dadurch gekennzeichnet, dass** die Konzentration der mineralischen Säure oder Sulfonsäure jeweils im Bereich von 1 bis 30 Gew.-%, bezogen auf das Reaktionsgemisch der Rückspaltung, beträgt.

6. Verfahren nach einem der vorhergehenden Ansprüche, **dadurch gekennzeichnet, dass** man die Umsetzung von Acrylsäure mit n-Butanol bei einer Temperatur im Bereich von 70 bis 160 °C und einem Absolutdruck in Bereich von 300 bis 1500 mbar durchführt.

7. Verfahren nach einem der vorhergehenden Ansprüche 1 bis 5, **dadurch gekennzeichnet, dass** man die Umsetzung von Acrylsäure mit n-Butanol bei einer Temperatur im Bereich von 80 bis 130 °C und einem Absolutdruck im Bereich von 400 bis 700 mbar durchführt.

8. Verfahren nach einem der vorhergehenden Ansprüche 1 bis 5, **dadurch gekennzeichnet, dass** man die Umsetzung von Acrylsäure mit n-Butanol bei einer Temperatur im Bereich von 110 bis 125 °C und einem Absolutdruck im Bereich von 500 bis 600 mbar durchführt.

9. Verfahren nach einem der vorhergehenden Ansprüche, **dadurch gekennzeichnet, dass** die Menge an zugesetzter Säure als Katalysator in die Reaktionszone im Bereich von 92 bis 153 mmol Säure pro kg des aus der Reaktionszone abgezogenen Reaktionsgemischs beträgt.

10. Verfahren nach einem der vorhergehenden Ansprüche 1 bis 8, **dadurch gekennzeichnet, dass** die Menge an zugesetzter Säure als Katalysator in die Reaktionszone im Bereich von 102 bis 143 mmol Säure pro kg des aus der Reaktionszone abgezogenen Reaktionsgemischs beträgt.

11. Verfahren nach einem der vorhergehenden Ansprüche, **dadurch gekennzeichnet, dass** man n-Butanol und Acrylsäure in einem Massenverhältnis im Bereich von n-Butanol : Acrylsäure = 1,05 bis 1,2 einsetzt.

12. Verfahren nach einem der vorhergehenden Ansprüche 1 bis 10, **dadurch gekennzeichnet, dass** man n-Butanol und Acrylsäure in einem Massenverhältnis im Bereich von n-Butanol : Acrylsäure = 1,1 bis 1,15 einsetzt.

13. Verfahren nach einem der vorhergehenden Ansprüche 2 bis 12, **dadurch gekennzeichnet, dass** die Rückspaltung VI des in der Rektifikationseinheit III anfallenden Sumpfprodukts bei einer Temperatur im Bereich von 120 bis 200 °C und einem Absolutdruck im Bereich von 400 bis 1500 mbar durchgeführt wird.

14. Verfahren nach einem der vorhergehenden Ansprüche 2 bis 12, **dadurch gekennzeichnet, dass** die Rückspaltung VI des in der Rektifikationseinheit III anfallenden Sumpfprodukts bei einer Temperatur im Bereich von 130 bis 190 °C und einem Absolutdruck im Bereich von 600 bis 1200 mbar durchgeführt wird.

15. Verfahren nach einem der vorhergehenden Ansprüche 2 bis 12, **dadurch gekennzeichnet, dass** die Rückspaltung VI des in der Rektifikationseinheit III anfallenden Sumpfprodukts bei einer Temperatur im Bereich von 150 bis 180 °C und einem Absolutdruck im Bereich von 900 bis 1100 mbar durchgeführt wird.

16. Verfahren nach einem der vorhergehenden Ansprüche, **dadurch gekennzeichnet, dass** in der Acrylsäure-Extraktionseinheit VIII das Sumpfprodukt der Rektifikationseinheit IV als Extraktionsmittel verwendet wird.

17. Verfahren nach einem der vorhergehenden Ansprüche, **dadurch gekennzeichnet, dass** die Verweilzeit der Edukte in der Reaktionszone im Bereich von 0,25 bis 5 Stunden beträgt.

18. Verfahren nach einem der vorhergehenden Ansprüche, **dadurch gekennzeichnet, dass** der Restgehalt an Acrylsäure im Reaktionsgemisch, das aus der Reaktionszone abgezogen wird, ≤ 5,0 Gew.-% beträgt.

19. Verfahren nach einem der vorhergehenden Ansprüche 2 bis 18, **dadurch gekennzeichnet, dass** die Rückspaltung VI des in der Rektifikationseinheit III anfallenden Sumpfprodukts in einem Rührkessel oder einem Behälter mit außenliegendem Wärmeübertrager, jeweils in einer kontinuierlichen, diskontinuierlichen oder halbkontinuierlichen Fahrweise, durchgeführt wird.

20. Verfahren nach einem der vorhergehenden Ansprüche 2 bis 19, **dadurch gekennzeichnet, dass** die Rückspaltung VI des in der Rektifikationseinheit III anfallenden Sumpfprodukts unter Zugabe von Acrylsäure und/oder michaeloligomerer Acrylsäure im Bereich von 1 bis 20 Gew.-%, bezogen auf die Menge des in der Rektifikationseinheit III anfallenden Sumpfprodukts, durchgeführt wird.

21. Verfahren nach einem der vorhergehenden Ansprüche 2 bis 20, **dadurch gekennzeichnet, dass** das Destillat der Rektifikationseinheit V zumindest teilweise der n-Butanol-Extraktionseinheit VII zugeführt wird (Strom 6).

22. Verfahren nach einem der vorhergehenden Ansprüche, **dadurch gekennzeichnet, dass** in der Strippeinheit IX organische Komponenten mit Wasserdampf oder durch indirekte Beheizung vom Wasser getrennt und über den Kopf der Strippeinheit zur Rektifikationseinheit I zurückgeführt werden.

23. Verfahren nach einem der vorhergehenden Ansprüche, **dadurch gekennzeichnet, dass** man einen Teil der organischen Phase, die nach Phasentrennung des Destillats der Rektifikationseinheit I anfällt, über eine n-Butanol-Extraktionseinheit VII leitet, die dort anfallende wässrige Phase in eine anschließende Strippeinheit IX leitet und die dort zurückgewonnene organische Phase in die Rektifikationseinheit I zurückführt.

24. Verfahren nach einem der vorhergehenden Ansprüche zur Herstellung von Acrylsäure-n-butylester in einer Reinheit von ≥ 99,5 Gew.-% und mit einem Gehalt an Di-n-butyl-ether (DBE) von ≤ 400 Gew.-ppm.

25. Verfahren nach dem vorhergehenden Anspruch zur Herstellung von Acrylsäure-n-butylester mit einem Gehalt an n-Butylacetat von ≤ 50 Gew.-ppm.

26. Verfahren nach einem der vorhergehenden Ansprüche 2 bis 20 zur Herstellung von Acrylsäure-n-butylester in einer Reinheit von ≥ 99,5 Gew.-% und mit einem Gehalt an Di-n-butyl-ether (DBE) von ≤ 200 Gew.-ppm.

27. Verfahren nach einem der vorhergehenden Ansprüche, **dadurch gekennzeichnet, dass** es sich bei der Säure als Veresterungskatalysator um eine schwefelhaltige mineralische Säure, phosphorhaltige mineralische Säure, organische Sulfonsäure, oder einer Mischung daraus handelt.

28. Verfahren nach dem vorhergehenden Anspruch, **dadurch gekennzeichnet, dass** es sich bei der Säure um Schwefelsäure, n-Butyl-schwefelsäure, aromatische Sulfonsäure, aliphatische Sulfonsäure, oder einer Mischung daraus handelt.

29. Verfahren nach dem vorhergehenden Anspruch, **dadurch gekennzeichnet, dass** es sich bei der Sulfonsäure um p-Toluolsulfonsäure, Methansulfonsäure, Benzolsulfonsäure, Dodecylbenzolsulfonsäure, Maranil^{®} DBS/LC (CAS-Nummer: 85536-14-7) oder einer Mischung daraus handelt.

## Claims

1. A process for continuously preparing n-butyl acrylate by reacting acrylic acid with n-butanol in a solvent-free phase at elevated temperature and with addition of acid as esterification catalyst, in which the acrylic acid, the n-butanol and the esterification catalyst are fed to a reaction zone, the water formed is separated from the reaction mixture during a dwell time in the reaction zone as a constituent of an n-butanol-comprising mixture in a first rectification unit I atop the reaction zone, the distillate obtained is separated into an n-butanol-comprising organic phase and a water-comprising aqueous phase, the organic phase is recycled to the rectification unit I, the aqueous phase is optionally sent wholly or partly to a stripping unit IX, the n-butyl acrylate-comprising reaction mixture which is drawn off from the reaction zone is sent to a prepurification, wherein
1. in a first prepurification stage (prepurification I), the predominant portion of the esterification catalyst is removed by extraction by water scrubbing and
2. in a second prepurification stage (prepurification II), the acidic components are neutralized and extracted with an aqueous alkali solution by reactive extraction and
3. optionally, in a third prepurification stage (prepurification III), residual salts and aqueous extraneous phase components are removed by extraction with water from the residual organic reaction mixture remaining after the second prepurification stage,
the remaining residual organic reaction mixture I is directed into a further separation zone comprising rectification units and the n-butyl ester of acrylic acid formed is separated off therein by
- feeding the remaining residual reaction mixture I to a rectification unit II and rectificatively separating the remaining residual reaction mixture I therein into a low boiler product comprising n-butyl acrylate and lower-boiling constituents than n-butyl acrylate, and a residual reaction mixture II comprising the n-butyl acrylate and higher-boiling constituents than n-butyl acrylate,
- feeding the residual reaction mixture II to a rectification unit III and separating the n-butyl acrylate therein from the higher-boiling constituents than n-butyl acrylate,
wherein
- the amount of acid added as catalyst to the reaction zone is in the range from 51 to 163 mmol of acid per kg of the reaction mixture drawn off from the reaction zone,
- n-butanol and acrylic acid are used in a mass ratio in the range of n-butanol:acrylic acid = 1.0 to 1.3,
- at least the organic component of the low boiler product from the rectification unit II that has not been used as return stream to the rectification unit II is fed at least partly to a rectification unit IV (stream 1) in which water and n-butanol and n-butyl acetate and di-n-butyl ether are distilled off, the distillate (stream 3) is fed at least partly to an n-butanol extraction unit VII, and the bottom product (stream 2) is recycled into the reaction zone either directly or via an acrylic acid extraction unit VIII.

2. The process according to claim 1, wherein
- the bottom product obtained in the rectification unit III is subjected to a redissociation VI in the presence of a sulfur-containing mineral acid, phosphorus-containing mineral acid or organic sulfonic acid at elevated temperature, and the redissociation products that escape in gaseous form are first condensed and then sent to a rectification unit V (stream 4) in which water, n-butanol and di-n-butyl ether are distilled off, and from which the bottom product is recycled into the reaction zone (stream 5).

3. The process according to the preceding claim, wherein the organic sulfonic acid is an aromatic sulfonic acid.

4. The process according to the preceding claim, wherein the aromatic sulfonic acid is dodecylbenzenesulfonic acid or Maranil^{®} DBS/LC (CAS number: 85536-14-7).

5. The process according to any of the preceding claims 2 to 4, wherein the concentration of the mineral acid or sulfonic acid is in each case in the range from 1% to 30% by weight, based on the redissociation reaction mixture.

6. The process according to any of the preceding claims, wherein the reaction of acrylic acid with n-butanol is conducted at a temperature in the range from 70 to 160°C and an absolute pressure in the range from 300 to 1500 mbar.

7. The process according to any of the preceding claims 1 to 5, wherein the reaction of acrylic acid with n-butanol is conducted at a temperature in the range from 80 to 130°C and an absolute pressure in the range from 400 to 700 mbar.

8. The process according to any of the preceding claims 1 to 5, wherein the reaction of acrylic acid with n-butanol is conducted at a temperature in the range from 110 to 125°C and an absolute pressure in the range from 500 to 600 mbar.

9. The process according to any of the preceding claims, wherein the amount of acid added as catalyst to the reaction zone is in the range from 92 to 153 mmol of acid per kg of the reaction mixture drawn off from the reaction zone.

10. The process according to any of the preceding claims 1 to 8, wherein the amount of acid added as catalyst to the reaction zone is in the range from 102 to 143 mmol of acid per kg of the reaction mixture drawn off from the reaction zone.

11. The process according to any of the preceding claims, wherein n-butanol and acrylic acid are used in a mass ratio in the range of n-butanol:acrylic acid = 1.05 to 1.2.

12. The process according to any of the preceding claims 1 to 10, wherein n-butanol and acrylic acid are used in a mass ratio in the range of n-butanol:acrylic acid = 1.1 to 1.15.

13. The process according to any of the preceding claims 2 to 12, wherein the redissociation VI of the bottom product obtained in the rectification unit III is conducted at a temperature in the range from 120 to 200°C and an absolute pressure in the range from 400 to 1500 mbar.

14. The process according to any of the preceding claims 2 to 12, wherein the redissociation VI of the bottom product obtained in the rectification unit III is conducted at a temperature in the range from 130 to 190°C and an absolute pressure in the range from 600 to 1200 mbar.

15. The process according to any of the preceding claims 2 to 12, wherein the redissociation VI of the bottom product obtained in the rectification unit III is conducted at a temperature in the range from 150 to 180°C and an absolute pressure in the range from 900 to 1100 mbar.

16. The process according to any of the preceding claims, wherein the bottom product from the rectification unit IV is used as extractant in the acrylic acid extraction unit VIII.

17. The process according to any of the preceding claims, wherein the dwell time of the reactants in the reaction zone is in the range from 0.25 to 5 hours.

18. The process according to any of the preceding claims, wherein the residual acrylic acid content in the reaction mixture which is drawn off from the reaction zone is ≤ 5.0% by weight.

19. The process according to any of the preceding claims 2 to 18, wherein the redissociation VI of the bottom product obtained in the rectification unit III is conducted in a stirred tank or a vessel with an external heat transferrer, in each case in a continuous, discontinuous or semicontinuous mode of operation.

20. The process according to any of the preceding claims 2 to 19, wherein the redissociation VI of the bottom product obtained in the rectification unit III is conducted with addition of acrylic acid and/or Michael acrylic acid oligomers in the range from 1% to 20% by weight, based on the amount of the bottom product obtained in the rectification unit III.

21. The process according to any of the preceding claims 2 to 20, wherein the distillate from the rectification unit V is sent at least partly to the n-butanol extraction unit VII (stream 6).

22. The process according to any of the preceding claims, wherein organic components are separated from water in the stripping unit IX with steam or by indirect heating and recycled to the rectification unit I via the top of the stripping unit.

23. The process according to any of the preceding claims, wherein a portion of the organic phase obtained after phase separation of the distillate from the rectification unit I is passed through an n-butanol extraction unit VII, the aqueous phase obtained therein is directed into a downstream stripping unit IX, and the organic phase recovered therein is recycled to the rectification unit I.

24. The process according to any of the preceding claims for preparing n-butyl acrylate in a purity of ≥ 99.5% by weight and with a di-n-butyl ether (DBE) content of ≤ 400 ppm by weight.

25. The process according to the preceding claim for preparing n-butyl acrylate with an n-butyl acetate content of ≤ 50 ppm by weight.

26. The process according to any of the preceding claims 2 to 20 for preparing n-butyl acrylate in a purity of ≥ 99.5% by weight and with a di-n-butyl ether (DBE) content of ≤ 200 ppm by weight.

27. The process according to any of the preceding claims, wherein the acid as esterification catalyst is a sulfur-containing mineral acid, phosphorus-containing mineral acid, organic sulfonic acid, or a mixture thereof.

28. The process according to the preceding claim, wherein the acid is sulfuric acid, n-butylsulfuric acid, aromatic sulfonic acid, aliphatic sulfonic acid, or a mixture of these.

29. The process according to the preceding claim, wherein the sulfonic acid is p-toluenesulfonic acid, methanesulfonic acid, benzenesulfonic acid, dodecylbenzenesulfonic acid, Maranil^{®} DBS/LC (CAS number: 85536-14-7) or a mixture thereof.

## Revendications

1. Procédé pour la préparation continue d'ester n-butylique de l'acide acrylique par transformation d'acide acrylique avec du n-butanol dans une phase exempte de solvant à température augmentée et avec addition d'acide en tant que catalyseur d'estérification, dans lequel on introduit l'acide acrylique, le n-butanol et le catalyseur d'estérification dans une zone de réaction, on sépare du mélange réactionnel, dans une première unité de rectification I placée sur la zone de réaction, pendant une durée de séjour dans la zone de réaction, l'eau formée en tant que constituant d'un mélange comprenant du n-butanol, on sépare le distillat ainsi obtenu en une phase organique contenant du n-butanol et en une phase aqueuse contenant de l'eau, on recycle la phase organique dans l'unité de rectification I, on recycle la phase aqueuse le cas échéant, totalement ou partiellement, dans une unité d'extraction IX, on introduit le mélange réactionnel contenant l'ester n-butylique de l'acide acrylique, qui est soutiré de la zone de réaction, dans une prépurification, où
1. dans une première étape de prépurification (prépurification I), on sépare la partie principale du catalyseur d'estérification par extraction au moyen d'un lavage à l'eau et
2. dans une deuxième étape de prépurification (prépurification II), on neutralise les composants acides avec une solution alcaline aqueuse par extraction réactive et on les extrait et
3. éventuellement, dans une troisième étape de prépurification (prépurification III), on élimine du mélange réactif résiduel organique restant après la deuxième étape de prépurification, les sels résiduels ainsi que les proportions aqueuses de phase étrangère par extraction avec de l'eau,
on guide le mélange réactionnel résiduel organique I restant dans une autre zone de séparation comprenant des unités de rectification et on sépare dans celle-ci l'ester n-butylique de l'acide acrylique formé en ce que
- on introduit le mélange réactionnel résiduel I qui reste dans une unité de rectification II et on sépare dans celle-ci par rectification le mélange réactionnel résiduel I qui reste en un produit de bas point d'ébullition contenant de l'ester n-butylique de l'acide acrylique et des constituants présentant un point d'ébullition plus bas que celui de l'ester n-butylique de l'acide acrylique et en un mélange réactionnel résiduel II comprenant de l'ester n-butylique de l'acide acrylique et des constituants présentant un point d'ébullition plus élevé que celui de l'ester n-butylique de l'acide acrylique,
- on introduit le mélange réactionnel résiduel II dans une unité de rectification III et on sépare dans celle-ci l'ester n-butylique de l'acide acrylique des constituants présentant un point d'ébullition plus élevé que celui de l'ester n-butylique de l'acide acrylique,
**caractérisé en ce que**
- la quantité d'acide ajouté en tant que catalyseur dans la zone de réaction se situe dans la plage de 51 à 163 mmoles d'acide par kg de mélange réactionnel soutiré de la zone de réaction.
- on utilise le n-butanol et l'acide acrylique dans un rapport massique dans la plage de n-butanol: acide acrylique = 1,0 à 1,3,
- on introduit au moins la proportion organique du produit à bas point d'ébullition non utilisé comme reflux dans l'unité de rectification II, provenant de l'unité de rectification II, au moins partiellement dans une unité de rectification IV (flux 1), dans laquelle de l'eau et du n-butanol et de l'acétate de n-butyle et du di-n-butyléther sont éliminés par distillation, on introduit le distillat (flux 3) au moins partiellement dans une unité d'extraction de n-butanol VII et on recycle le produit de fond (flux 2) soit directement soit par l'intermédiaire d'une unité d'extraction d'acide acrylique VIII dans la zone de réaction.

2. Procédé selon la revendication 1, **caractérisé en ce que**
- le produit de fond obtenu dans l'unité de rectification III est soumis à une dissociation en retour VI en présence d'un acide minéral contenant du soufre, d'un acide minéral contenant du phosphore ou d'un acide sulfonique organique à température augmentée et les produits de dissociation en retour qui se dégagent sous forme gazeuse sont d'abord condensés puis introduits dans une unité de rectification V (flux 4), dans laquelle de l'eau, du n-butanol et du di-n-butyléther sont éliminés par distillation et dont le produit de fond est recyclé dans la zone de réaction (flux 5).

3. Procédé selon la revendication précédente, **caractérisé en ce qu'**il s'agit, pour l'acide sulfonique organique, d'un acide sulfonique aromatique.

4. Procédé selon la revendication précédente, **caractérisé en ce qu'**il s'agit, pour l'acide sulfonique aromatique, d'acide dodécylbenzènesulfonique ou de Maranil^{®} DBS/LC (numéro CAS : 85536-14-7).

5. Procédé selon l'une des revendications précédentes 2 à 4, **caractérisé en ce que** la concentration en acide minéral ou en acide sulfonique se situe à chaque fois dans la plage de 1 à 30% en poids, par rapport au mélange réactionnel de la dissociation en retour.

6. Procédé selon l'une des revendications précédentes, **caractérisé en ce qu'**on effectue la transformation de l'acide acrylique avec du n-butanol à une température dans la plage de 70 à 160°C et à une pression absolue dans la plage de 300 à 1500 mbars.

7. Procédé selon l'une des revendications 1 à 5, **caractérisé en ce qu'**on effectue la transformation de l'acide acrylique avec du n-butanol à une température dans la plage de 80 à 130°C et à une pression absolue dans la plage de 400 à 700 mbars.

8. Procédé selon l'une des revendications 1 à 5, **caractérisé en ce qu'**on effectue la transformation de l'acide acrylique avec du n-butanol à une température dans la plage de 110 à 125°C et à une pression absolue dans la plage de 500 à 600 mbars.

9. Procédé selon l'une des revendications précédentes, **caractérisé en ce que** la quantité d'acide ajouté en tant que catalyseur dans la zone de réaction se situe dans la plage de 92 à 153 mmoles d'acide par kg de mélange réactionnel soutiré de la zone de réaction.

10. Procédé selon l'une des revendications précédentes 1 à 8, **caractérisé en ce que** la quantité d'acide ajouté en tant que catalyseur dans la zone de réaction se situe dans la plage de 102 à 143 mmoles d'acide par kg de mélange réactionnel soutiré de la zone de réaction.

11. Procédé selon l'une des revendications précédentes, **caractérisé en ce qu'**on utilise le n-butanol et l'acide acrylique dans un rapport massique dans la plage de n-butanol: acide acrylique = 1,05 à 1,2.

12. Procédé selon l'une des revendications précédentes 1 à 10, **caractérisé en ce qu'**on utilise le n-butanol et l'acide acrylique dans un rapport massique dans la plage de n-butanol: acide acrylique = 1,1 à 1,15.

13. Procédé selon l'une des revendications 2 à 12, **caractérisé en ce qu'**on effectue la dissociation en retour VI du produit de fond obtenu dans l'unité de rectification III à une température dans la plage de 120 à 200°C et à une pression absolue dans la plage de 400 à 1500 mbars.

14. Procédé selon l'une des revendications 2 à 12, **caractérisé en ce qu'**on effectue la dissociation en retour VI du produit de fond obtenu dans l'unité de rectification III à une température dans la plage de 130 à 190°C et à une pression absolue dans la plage de 600 à 1200 mbars.

15. Procédé selon l'une des revendications 2 à 12, **caractérisé en ce qu'**on effectue la dissociation en retour VI du produit de fond obtenu dans l'unité de rectification III à une température dans la plage de 150 à 180°C et à une pression absolue dans la plage de 900 à 1100 mbars.

16. Procédé selon l'une des revendications précédentes, **caractérisé en ce qu'**on utilise, dans l'unité d'extraction d'acide acrylique VIII, le produit de fond de l'unité de rectification VI en tant qu'agent d'extraction.

17. Procédé selon l'une des revendications précédentes, **caractérisé en ce que** la durée de séjour des produits de départ dans la zone de réaction se situe dans la plage de 0,25 à 5 heures.

18. Procédé selon l'une des revendications précédentes, **caractérisé en ce que** la teneur résiduelle en acide acrylique dans le mélange réactionnel qui est soutiré de la zone de réaction, est ≤ 5,0% en poids.

19. Procédé selon l'une des revendications précédentes 2 à 18, **caractérisé en ce que** la dissociation en retour VI du produit de fond obtenu dans l'unité de rectification III est effectuée dans une cuve agitée ou dans un récipient présentant un dispositif externe de transfert de chaleur, à chaque fois dans un mode opératoire continu, discontinu ou semi-continu.

20. Procédé selon l'une des revendications précédentes 2 à 19, **caractérisé en ce que** la dissociation en retour VI du produit de fond obtenu dans l'unité de rectification III est effectuée avec addition d'acide acrylique et/ou d'acide acrylique oligomère selon Michael dans la plage de 1 à 20% en poids, par rapport à la quantité du produit de fond obtenu dans l'unité de rectification III.

21. Procédé selon l'une des revendications précédentes 2 à 20, **caractérisé en ce que** le distillat de l'unité de rectification V est introduit au moins partiellement dans l'unité d'extraction de n-butanol VII (flux 6).

22. Procédé selon l'une des revendications précédentes, **caractérisé en ce que**, dans l'unité d'extraction IX, les composants organiques sont séparés de l'eau à l'aide de vapeur d'eau ou par chauffage indirect et introduits par l'intermédiaire de la tête de l'unité d'extraction dans l'unité de rectification I.

23. Procédé selon l'une des revendications précédentes, **caractérisé en ce que** l'on guide une partie de la phase organique, qui est obtenue après la séparation des phases du distillat de l'unité de rectification I, sur une unité d'extraction de n-butanol VII, on guide la phase aqueuse qui y est obtenue dans une unité d'extraction IX consécutive et on recycle la phase organique qui y est récupérée dans l'unité de rectification I.

24. Procédé selon l'une des revendications précédentes pour la préparation d'ester n-butylique de l'acide acrylique à une pureté ≥ 99,5% en poids et présentant une teneur en di-n-butyléther (DBE) ≤ 400 ppm en poids.

25. Procédé selon la revendication précédente pour la préparation d'ester n-butylique de l'acide acrylique présentant une teneur en acétate de n-butyle ≤ 50 ppm en poids.

26. Procédé selon l'une des revendications précédentes 2 à 20 pour la préparation d'ester n-butylique de l'acide acrylique à une pureté ≥ 99,5% en poids et présentant une teneur en di-n-butyléther (DBE) ≤ 200 ppm en poids.

27. Procédé selon l'une des revendications précédentes, **caractérisé en ce qu'**il s'agit, pour l'acide en tant que catalyseur d'estérification, d'un acide minéral contenant du soufre, d'un acide minéral contenant du phosphore, d'un acide sulfonique organique ou d'un mélange de ceux-ci.

28. Procédé selon la revendication précédente, **caractérisé en ce qu'**il s'agit, pour l'acide, d'acide sulfurique, d'acide n-butylsufurique, d'acide sulfonique aromatique, d'acide sulfonique aliphatique ou d'un mélange de ceux-ci.

29. Procédé selon la revendication précédente, **caractérisé en ce qu'**il s'agit, pour l'acide sulfonique, d'acide p-toluènesulfonique, d'acide méthanesulfonique, d'acide benzènesulfonique, d'acide dodécylbenzènesulfonique, de Maranil^{®} DBS/LC (numéro CAS : 85536-14-7) ou d'un mélange de ceux-ci.
